# EUROPEAN PATENT SPECIFICATION

(11) **EP 1 272 204 B1**
(45) Date of publication and mention of the grant of the patent: **20.06.2007**
(21) Application number: 01924998.6
(22) Date of filing: 12.04.2001
(51) Int. Cl.: A61K 35/34, A61P 43/00

(54) **SOFT TISSUE AND BONE AUGMENTATION AND BULKING UTILIZING MUSCLE-DERIVED PROGENITOR CELLS, COMPOSITIONS AND TREATMENTS THEREOF**
VERMEHRUNG DES WEICH- SOWIE DES KNOCHENGEWEBES ANHAND VON AUS MUSKELN STAMMENDEN VORLÄUFERZELLEN, SOWIE DAMIT VERBUNDENE ZUSAMMENSETZUNGEN UND BEHANDLUNGSFORMEN
AUGMENTATION ET ETOFFEMENT DE TISSUS MOUS ET OSSEUX AU MOYEN DE CELLULES SOUCHES DERIVEES DE MUSCLES, COMPOSITIONS CONTENANT CES CELLULES ET PROCEDES ASSOCIES

(30) Priority: 14.04.2000 US 549937
(43) Date of publication of application: 08.01.2003
(73) Proprietor: UNIVERSITY OF PITTSBURGH, Pittsburgh, PA 15260 (US)
(72) Inventor: Chancellor, Michael B., Pittsburgh, PA 15217 (US); Huard, Johnny, Waxford, PA 15090 (US); Capelli, Christopher C., Pittsburgh, PA 15217 (US); Qu, Zhuqing, Pittsburg, PA 15232 (US)
(74) Representative: Killin, Stephen James
(86) International application number: PCT/US2001/012084
(87) International publication number: WO 2001/078754

(56) References cited:
- WO-A-99/56785
- QU ZHUQING ET AL: "Identification of muscle-derived stem cells." MOLECULAR BIOLOGY OF THE CELL, vol. 10, no. SUPPL., November 1999 (1999-11), page 246a XP000982555 39th Annual Meeting of the American Society for Cell Biology;Washington, D.C., USA; December 11-15, 1999 ISSN: 1059-1524
- SMITH J ET AL: "STABLE INTREGRATION OF AN MDX SKELETAL MUSCLE CELL LINE INTO DYSTROPHIC (MDX) SKELETAL MUSCLE: EVIDENCE FOR STEM CELL STATUS" CELL GROWTH AND DIFFERENTIATION, XX, XX, vol. 8, no. 8, August 1997 (1997-08), pages 927-934, XP000982572
- JANKOWSKI RON J ET AL: "Flow cytometric characterization of myogenic cell populations obtained via the preplate technique: Potential for rapid isolation of muscle-derived stem cells." HUMAN GENE THERAPY, vol. 12, no. 6, 10 April 2001 (2001-04-10), pages 619-628, XP002183830 ISSN: 1043-0342

## Description

The present invention relates to muscle-derived progenitor (or stem) cells (MDC or MDSC) and compositions of MDC and their use in the augmentation of body tissues, particularly soft tissue and bone. In particular, the present invention relates to muscle-derived progenitor cells that show long-term survival following introduction into soft tissues and bone, methods of isolating MDC, and methods of using MDC-containing compositions for the augmentation of human or animal soft tissues and bone, including epithelial, adipose, nerve, organ, muscle, ligament, and cartilage tissue. The invention also relates to novel uses of muscle-derived progenitor cells for the treatment of cosmetic or functional conditions, such as dermatological conditions, gastroesophageal reflux, vesico-ureteral reflux, urinary incontinence, fecal incontinence, skeletal muscle weakness, heart failure, and injury or weakness associated with myocardial infarction.

Augmentation of soft tissue using synthetic materials such as silicone or polytetrafluoroethylene (PTFE) is well known in the art. U.S. Patent No. 5,876,447 to Amett discloses the use of silicone implants for facial plastic surgery. However, such synthetic materials are foreign to the host tissue, and cause an Immunological response resulting in the encapsulation of the Implant and scarring of the surrounding tissues. Thus, the Implant may produce additional functional or aesthetic problems.

Soft tissue augmentation using biopolymers such as collagen or hyaluronic acid has also been described. For example, U.S. Patent No. 4,424,208 to Wallace et al. discloses methods of augmenting soft tissue utilizing collagen implant material. In addition, U.S. Patent No. 4,965,353 to delta Valle et al. discloses esters of hyaluronic acid that can be used in cosmetic surgery. However, these biopolymers are also foreign to the host tissue, and cause an immunological response resulting in the reabsorption of the injected material. Biopolymers are therefore unable to provide long-term tissue augmentation. Overall, the use of biopolymers or synthetic materials has been wholly unsatisfactory for the purpose of augmenting soft tissue.

Soft tissue augmentation using cell-based compositions has also been developed. U.S. Patent No. 5,858,390 to Boss, Jr. discloses the use of autologous dermal fibroblasts for the treatment of cosmetic and aesthetic skin defects. Although this treatment avoids the problems inherent in the implantation or injection of synthetic materials or biopolymers, it results in other complications. Because fibroblasts produce collagen, the cells can cause the stiffening and distortion of the tissues surrounding the implant site.

The use of autologous fat cells as an Injectable bulking agent has also been described (For review, see K. Mak et al, 1994, Otolaryngol. Clin. North. Am.27:211-22;American Society of Plastic and Reconstructive Surgery: Report on autologous fat transplantation by the ad hoc committee on new procedures, 1987, Chicago: American Society of Plastic and Reconstructive Surgery*;* A. Chaichir et al., 1989, Plast. Reconstr. Surg. 84: 921-935; R.A. Ersek, 1991, Plast. Reconstr. Surg. 87:219-228; H.W. Horl et al., 1991, Ann. Plast. Surg. 26:248-258; A. Nguyen et al., 1990, Plast. Reconstr. Surg. 85:378-389; J. Sartynski et al., 1990, Otolaryngol. Head Neck Surg. 102:314-321. However, the fat grafting procedure provides only temporary augmentation, as injected fat is reabsorbed into the host. In addition, fat grafting can result in nodule formation and tissue asymmetry.

Myoblasts, the precursors of muscle fibers, are mononucleated muscle cells that fuse to form post-mitotic multinucleated myotubes, which can provide long-term expression and delivery of bioactive proteins (T.A. Partridge and K.E. Davies, 1995, Brit. Med. Bulletin 51:123-137; J. Dhawan et al., 1992, Science 254: 1509-12; A.D. Grinnell, 1994, Myology Ed 2, A.G. Engel and C.F. Armstrong, McGraw-Hill, Inc., 303-304; S. Jiao and J.A. Wolff, 1992, Brain Research 575:143-7; H. Vandenburgh, 1996, Human Gene Therapy 7:2195-2200).

Cultured myoblasts contain a subpopulation of cells that show some of the self-renewal properties of stem cells (A. Baroffio et al., 1996, Differentiation 60:47-57). Such cells fall to fuse to form myotubes, and do not divide unless cultured separately (A. Baroffio et al., *supra).* Studies of myoblast transplantation (see below) have shown that the majority of transplanted cells quickly die, while a minority survive and mediate new muscle formation (J.R. Beuchamp et al., 1999, J. Cell Biol. 144:1113-1122). This minority of cells shows distinctive behavior, including slow growth in tissue culture and rapid growth following transplantation, suggesting that these cells may represent myoblast stem cells (J.R. Beuchamp et al., *supra)*.

Myoblasts have been used as vehicles for gene therapy in the treatment of various muscle- and non-muscle-related disorders. For example, transplantation of genetically modified or unmodified myoblasts has been used for the treatment of Duchenne muscular dystrophy (E. Gussoni et al., 1992, Nature, 356:435-8; J. Huard et al., 1992, Muscle & Nerve, 15:550-60; G. Karpati et al., 1993, Ann. Neurol., 34:8-17; J.P. Tremblay et al., 1993, Cell Transplantation; 2:99-112; P.A. Moisset et al., 1998, Biochem. Biophys. Res. Commun. 247:94-9; P.A. Moisset et al., 1998, Gene Ther. 5:1340-46). In addition, myoblasts have been genetically engineered to produce proinsulin for the treatment of Type 1 diabetes (L. Gros et al., 1999, Hum. Gen. Ther. 10:1207-17); Factor IX for the treatment of hemophilia B (M. Roman et al., 1992, Somat. Cell. Mol. Genet. 18:247-58; S.N. Yao et al., 1994, Gen. Ther. 1:99-107; J.M. Wang et al., 1997, Blood 90:1075-82; G. Hortelano et al., 1999, Hum. Gene Ther. 10:1281-8); adenosine deaminase for the treatment of adenosine deaminase deficiency syndrome (C.M. Lynch et al., 1992, Proc. Natl. Acad. Sci. USA, 89:1138-42); erythropoietin for the treatment of chronic anemia (E. Regulier et al., 1998, Gene Ther. 5:1014-22; B. Dalle et al., 1999, Gene Ther. 6:157-61), and human growth hormone for the treatment of growth retardation (K. Anwer et al., 1998, Hum. Gen. Ther. 9:659-70).

Myoblasts have also been used to treat muscle tissue damage or disease, as disclosed in U.S. Patent No. 5,130,141 to Law et al., U.S. Patent No. 5,538,722 to Blau et al., and application U.S. Serial No. 09/302,896 filed April 30, 1999 by Chancellor et al. In addition, myoblast transplantation has been employed for the repair of myocardial dysfunction (C.E. Murry et al., 1996, J. Clin. Invest. 98:2512-23; B.Z. Atkins et al., 1999, Ann. Thorac. Surg. 67:124-129; B.Z. Atkins et al., 1999, J. Heart Lung Transplant 18:1173-80).

In spite of the above, In most cases, primary myoblast-derived treatments have been associated with low survival rates of the cells following transplantation due to migration and/or phagocytosis. To circumvent this problem, U.S. Patent 5,667;778 to Atala discloses the use of myoblasts suspended in a liquid polymer, such as alginate. The polymer solution acts as a matrix to prevent the myoblasts from migrating and/or undergoing phagocytosis after injection. However, the polymer solution presents the same problems as the biopolymers discussed above. Furthermore, the Atala patent is limited to uses of myoblasts in only muscle tissue, but no other tissue.

Thus, there is a need for other, different soft tissue augmentation materials that are long-lasting, compatible with a wide range of host tissues, and which cause minimal inflammation, scarring, and/or stiffening of the tissues surrounding the implant site. Accordingly, the muscle-derived progenitor cell-containing compositions of the present invention are provided as improved and novel materials for augmenting soft tissues. Further provided are methods of producing muscle-derived progenitor cell compositions that show long-term survival following transplantation, and methods of utilizing MDC and compositions containing MDC to treat various aesthetic and/or functional defects, including, for example, dermatological conditions or injury, and muscle weakness, injury, disease, or dysfunction.

It is notable that prior attempts to use myoblasts for non-muscle soft tissue augmentation were unsuccessful (U.S. Patent No. 5,667,778 to Atala). Therefore, the findings disclosed herein are unexpected, as they show that the muscle-derived progenitor cells according to the present invention can be successfully transplanted into non-muscle and muscle soft tissue, including epithelial tissue, and exhibit long-term survival. As a result, MDC and compositions comprising MDC can be used as a general augmentation material for muscle or non-muscle soft tissue augmentation, as well as for bone production. Moreover, since the muscle-derived progenitor cells and compositions of the present invention can be derived from autologous sources, they carry a reduced risk of immunological complications in the host, including the reabsorption of augmentation materials, and the inflammation and/or scarring of the tissues surrounding the implant site.

Although mesenchymal stem cells can be found In various connective tissues of the body including muscle, bone, cartilage, etc. (H.E. Young et al.,1993, in Vitro Cell Dev. Biol. 29A:723-736; H.E. Young, et al., 1995, Dev. Dynam. 202:137-144), the term mesenchymal has been used historically to refer to a class of stem cells purified from bone marrow, and not from muscle. Thus, mesenchymal stem cells are distinguished from the muscle-derived progenitor cells of the present Invention. Moreover, mesenchymal cells do not express the CD34 cell marker (M.F. Pittenger et ai, 1999, Science 284:143-147), which is expressed by the muscle-derived progenitor cells described herein.

A major problem for cell therapy is the poor survival and limited spread of the injected cells, as well as the immune rejection of recipients against donor cells (Y. Fan et al., 1996, Muscle & Nerve, 19:853-860). Muscle-derived stem cells (MDSC or MDC) as described by the present invention show the capacity of high self-renewal and long term proliferation when used in cell transplant therapies to augment and bulk soft tissue and bone. Both autologous and allogeneic cells of this invention can provide effective cell therapies for the disorders and conditions described. In addition, such cells can improve the efficiency of cell therapy in severe diseased muscles.

In a first aspect of the invention, there is provided, use of a composition comprising (i) isolated, desmin-expressing, muscle-derived progenitor cells (MDC) having long-term survivability *in situ,* or a clonal population thereof, and (ii) a physiologically-acceptable carrier, excipient, or diluent, for the manufacture of a medicament for use in augmenting or bulking esophagael muscle tissue or gastroesophageal muscle tissue in a mammal, wherein the composition is present in an amount sufficient to augment or bulk the esophageal or gastroesophageal muscle tissue.

According to a second aspect of the invention, there is provided use of a composition comprising (i) isolated, desmin-expressing, muscle-derived progenitor cells (MDC) having long-term survivability *in situ,* or a clonal population thereof, and (ii) a physiotogically-acceptable carrier, excipient, or diluent, for the manufacture of a medicament for use in augmenting or bulking sphincter muscle tissue in a mammal, wherein the composition is present in an amount sufficient to augment or bulk the sphincter muscle tissue.

In a third aspect of the present invention, there is provided use of a composition comprising (i) isolated, desmin-expressing, muscle-derived progenitor cells (MDC) having long-term survivability *in situ,* or a clonal population thereof, and (ii) a physiologically-acceptable carrier, excipient, or diluent, for the manufacture of a medicament for use in augmenting or bulking tissue selected from bladder, ureteral, or urethral muscle tissue in a mammal, wherein the composition is present in an amount sufficient to augment or bulk the bladder, ureteral, or urethral tissue.

According to a fourth aspect of the invention, there is provided use of a composition comprising (i) isolated, desmin-expressing, muscle-derived progenitor cells (MDC) having long-term survivability *in situ,* or a clonal population thereof, and (ii) a physiologically-acceptable carrier, excipient, or diluent, for the manufacture of a medicament for use in augmenting or bulking a soft tissue cosmetic or aesthetic defect in a mammal, wherein the composition is present in an amount sufficient to augment or bulk the cosmetic or aesthetic defect.

In a fifth aspect of the invention, there is provided use of a composition comprising (i) isolated, desmin-expressing, muscle-derived progenitor cells (MDC) having long-term survivability *in situ,* or a clonal population thereof, and (ii) a physiologically-acceptable carrier, excipient, or diluent, for the manufacture of a medicament for use in augmenting or bulking tissue comprising one or more of a cutaneous depression, wound, fissure, or opening in a mammal, wherein the composition is present in an amount sufficient to augment or bulk the cutaneous depression, wound, fissure, or opening.

According to a sixth aspect of the invention, there is provided use of a composition comprising (i) isolated, desmin-expressing, muscle-derived progenitor cells (MDC) having long-term survivability *in situ,* or a clonal population thereof, and (ii) a physiologically-acceptable carrier, excipient, or diluent, for the manufacture of a medicament for use in augmenting or bulking muscle tissue in a mammal, wherein the composition is present in an amount sufficient to augment or bulk the muscle tissue; said muscle tissue being selected from (a) digestive tissue selected from the group consisting of stomach, intestinal, tongue, esophageal, pyloric and anal tissue; (b) reproductive tissue selected from the group consisting of uterine, vaginal, clitoral, fallopian tube, cervical, penile and vas deferens tissue; (c) urological tissue selected from the group consisting of kidney, bladder, urethral, ureteral and sphincter tissue; or (d) respiratory tissue selected from the group consisting of tracheal and lung tissue.

In a seventh aspect of the invention, there is provided use of a composition comprising (i) isolated, desmin-expressing, muscle-derived progenitor cells (MDC) having long-term survivability *in situ,* or a clonal population thereof, and (ii) a physiologically-acceptable carrier, excipient, or diluent, for the manufacture of a medicament for use in augmenting or bulking (a) non-muscle soft tissue, or (b) non-muscle, non-bone soft tissue in a mammal, wherein the composition is present in an amount sufficient to augment or bulk the (a) non-muscle soft tissue, or (b) the non-muscle, non-bone soft tissue.

According to an eighth aspect of the invention, there is provided use of a composition comprising (i) isolated, desmin-expressing, muscle-derived progenitor cells (MDC) having long-term survivability *in situ,* or a clonal population thereof, and (ii) a physiologically-acceptable carrier, excipient, or diluent, for the manufacture of a medicament for use in restoring or improving contractibility of smooth muscle, wherein the smooth muscle is gastrointestinal smooth muscle selected from the group consisting of eosphagus smooth muscle, stomach smooth muscle, pyloric smooth muscle, and intestine smooth muscle; and further wherein the composition is present in an amount sufficient to restore or improve the smooth muscle contractility.

Preferred embodiments of the invention in any of its various aspects are as described below or as defined in the sub claims.

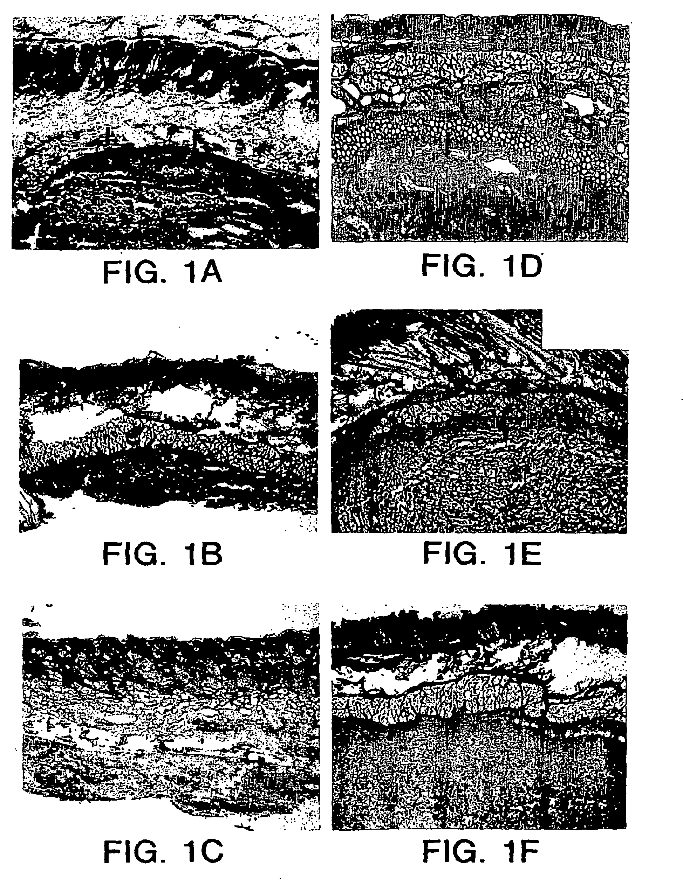

**Figures 1A-1F** illustrate the results of soft tissue augmentation utilizing injections of MDC compositions compared with injection of conventional bovine collagen. For Figures 1A-1F, either MDC (Figures 1D-1F) or collagen (1A-1C) were injected into the skin of the abdominal wall. The area of injection was the interface of the dermis and the subcutaneous connective tissue which is the skin. Figures 1A-1F show Trichrome staining at 40 x magnification following injection of either collagen or MDC into skin. At 5 days, 2 weeks, or 4 weeks post-injection, tissue samples were obtained and prepared for analysis. Figures 1A and 1D show the results of MDC versus collagen injection into the skin at 5 days post-injection; Figures 1B and 1E show the results at 2 weeks post-injection; and Figures 1C and 1F show the results at 4 weeks post-injection. Arrowheads in Figures 1D-1F indicate the presence of MDC in the injected areas (deep pink color). Figures 1A-1 F demonstrate that following injection into the subcutaneous space, MDC persisted and maintained/augmented the abdominal wall subcutaneous tissue for up to at least 4 weeks, while collagen did not persist by 2 weeks post-injection into the skin. (Example 3).

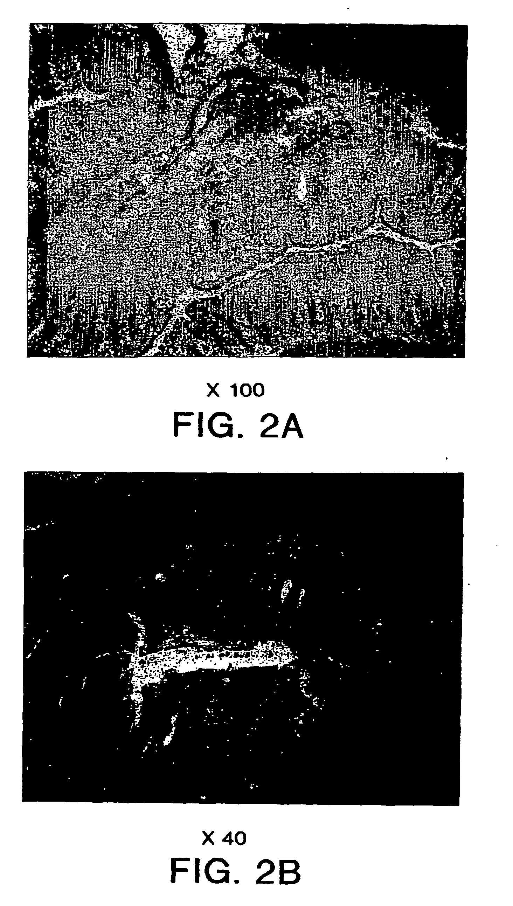

**Figures 2A** and **2B** illustrate the results of lower esophageal (Figure 2A) and anal sphincter (Figure 2B) soft tissue augmentation utilizing injections of MDC compositions. Injections were made Into the gastroesophageal junction or anal sphincter At day 3 post-injection, tissue samples were obtained and prepared for analysis. MDC are indicated by β-galactosidase staining. Figure 2A shows injected tissues at 100 X magnification; Figure 2B shows injected tissues at 40 X magnification. Figures 2A and 2B demonstrates that MDC injections maintained the lower esophageal sphincter and anal sphincter soft tissue augmentation for up to 3 days following injection

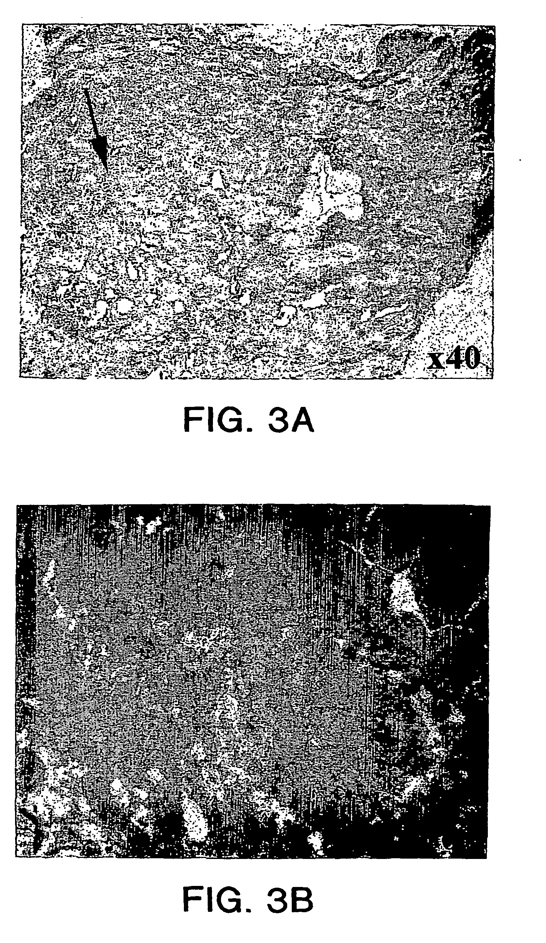

**Figures 3A** and **3B** illustrate the results of bladder-ureteral junction soft tissue augmentation utilizing injections of MDC compositions. Injections were made into the vesico-ureteral junction. At day 3 post-injection, tissue samples were obtained and prepared for analysis. MDC are indicated by β-galactosidase staining as viewed near the arrow. Figure 3A shows injected tissues at low (40 X) magnification; Figure 3B shows injected tissues at high (100 X) magnification. Figures 3A and 3B demonstrate that MDC injections maintained the bladder-ureteral junction soft tissue augmentation for up to 3 days following injection

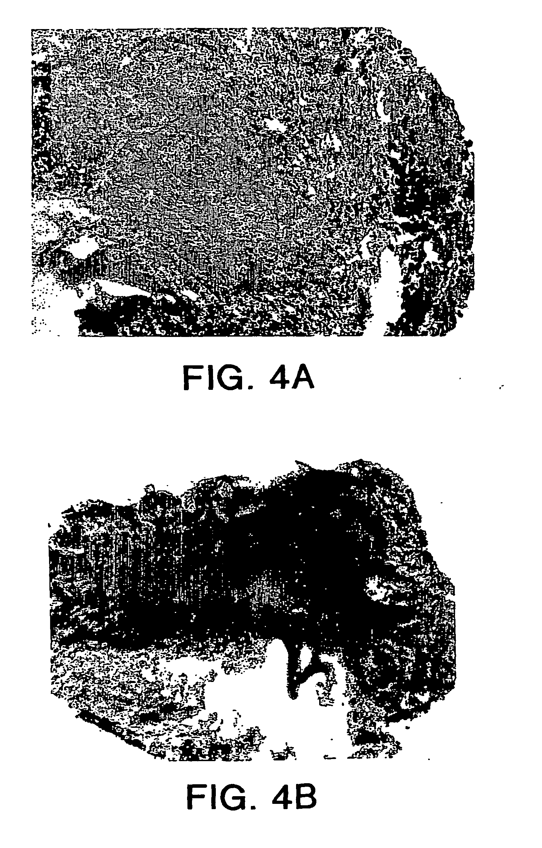

**Figures 4A** and **4B** Illustrate the treatment of bladder cryoinjury utilizing soft tissue injections of MDC compositions. Injections were made Into the bladder wall at the site of cryoinjury. At day 30 post-injection, tissue samples were obtained and prepared for staining. Arrows indicate site of cryoinjury and MDC injection. Magnification is 100 X. Figure 4A shows untreated cryodamaged bladder tissue. Figure 4B shows cryodamaged bladder tissue treated with MDC injections; MDC are indicated by β-galactosidase staining. Figures 4A and 4B demonstrate that MDC injections maintained the soft tissue augmentation of the cryodamaged bladder tissue for up to 30 days following injection.

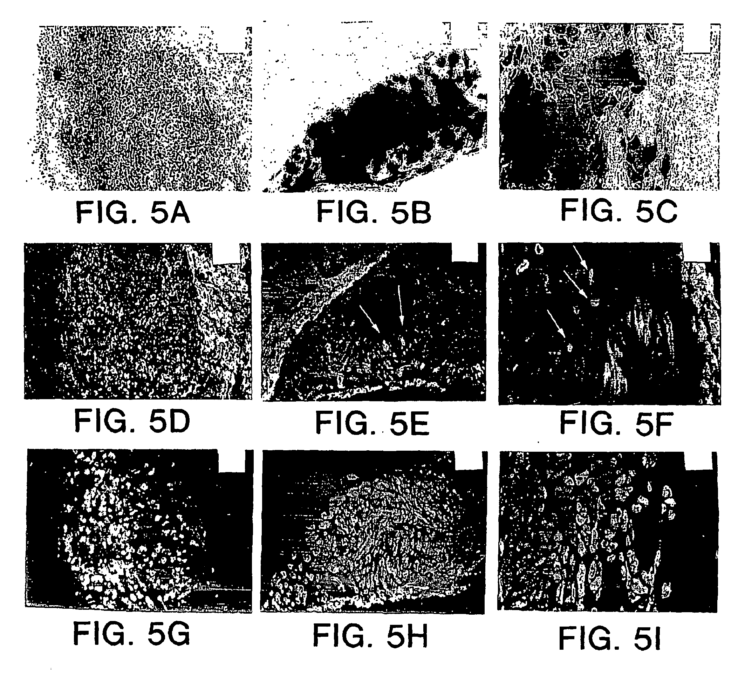

**Figures 5A-5I** Illustrate cellular differentiation of MDC following injection into cryodamaged bladder tissue. Injections were made into the bladder wall at the site of cryoinjury, and tissue samples were obtained and prepared for analysis at 5, 35, or 70 days post-injection, injected MDC are shown by staining for β-galactosidase, and undifferentiated MDC are shown by α-smooth muscle actin (α-SM actin) staining. MDC that have differentiated into myotubes or myofibers are shown by fast myosin heavy chain (fast MyHC) staining. Arrows show fast MyHC. At day 5 post-injection, multiple MDC are observed at the injection area and only some MDC have differentiated into myotubes, as shown by the high levels of β-galactosidase, (Figure 5A) and α-SM actin (Figure 5D) staining, and the relatively low levels of Fast MyHC (Figure 5G) staining. At day 35 post-injection, multiple MDC are observed at the injection area, and many have differentiated into myotubes, as shown by the high levels of β-galactosidase staining (Figure 5B), the decrease in α-SM actin (Figure 5E) staining; and the increase in Fast MyHC (Figure 5H) staining. At day 70 post-injection. MDC are observed at the injection area, and almost all MDC have differentiated into myofibers, as shown by the high levels of β-galactosidase (Figure 5C), the decrease in α-SM actin (Figure 5F) staining, and the high levels of Fast MyHC (Figure 51) staining. Magnification is 200 X. Figures 5A-5I demonstrate that MDC remain viable and begin differentiation for up to 70 days following injection into bladder soft tissue.

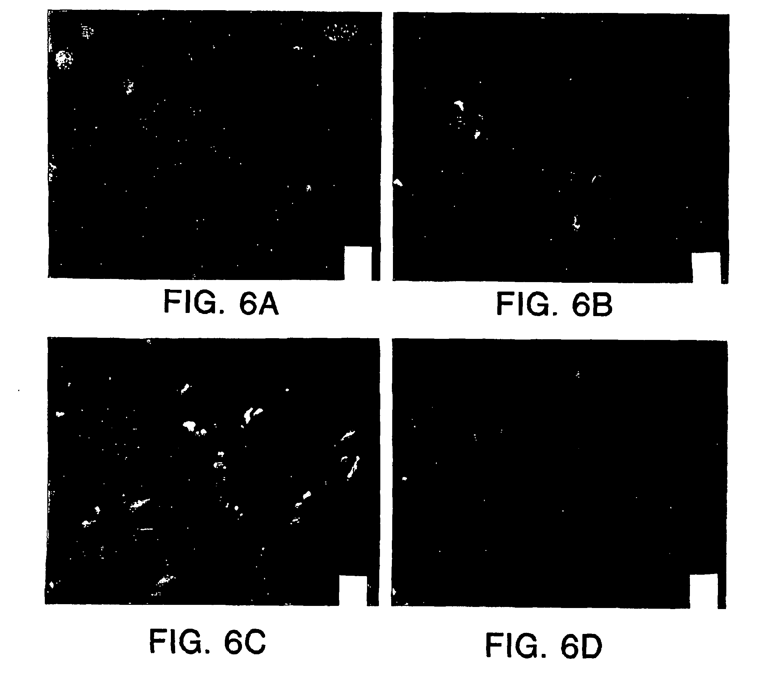

**Figures 6A-6D** illustrate the reinnervation of MDC injected into the soft tissue of the urinary bladder. Innervation is Indicated by acetylcholine (Ach) staining, which shows the neuromuscular junction. At day 3 post-injection, few innervations are observed, as shown by Ach staining (Figure 6A). At day 15 post injection, several innervations are observed (Figure 6B). At day 30 post-injection, more innervations are observed (Figure 6C). After 6 months post-injection, numerous innervations are observed at low (100 X) magnification (Figure 6D). Figures 6A-6C show injected tissue at high (200 X) magnification. Figures 6A-6D demonstrate that MDC induce innervation for up to 6 months following injection into cryodamaged bladder tissues.

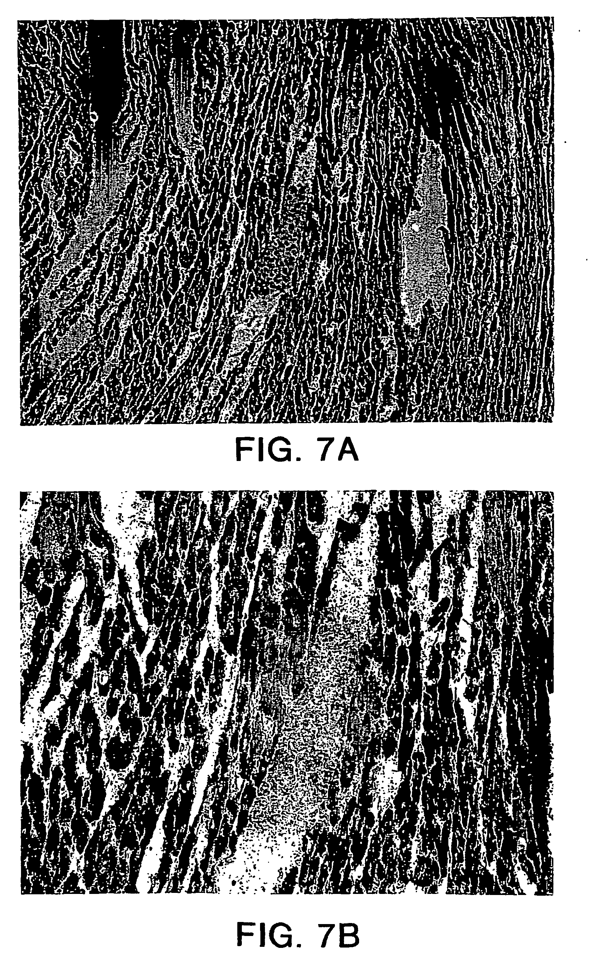

**Figures 7A** and **7B** illustrate the results of soft tissue augmentation of myocardial smooth muscle utilizing injections of MDC compositions, injections were made into the ventricular wall, and tissue samples were prepared 3 days post-injection. MDC are indicated by β-galactosidase staining. Figure 7A shows injected tissue at low (100X) magnification; Figure 7B shows injected tissue at high (200 X) magnification.

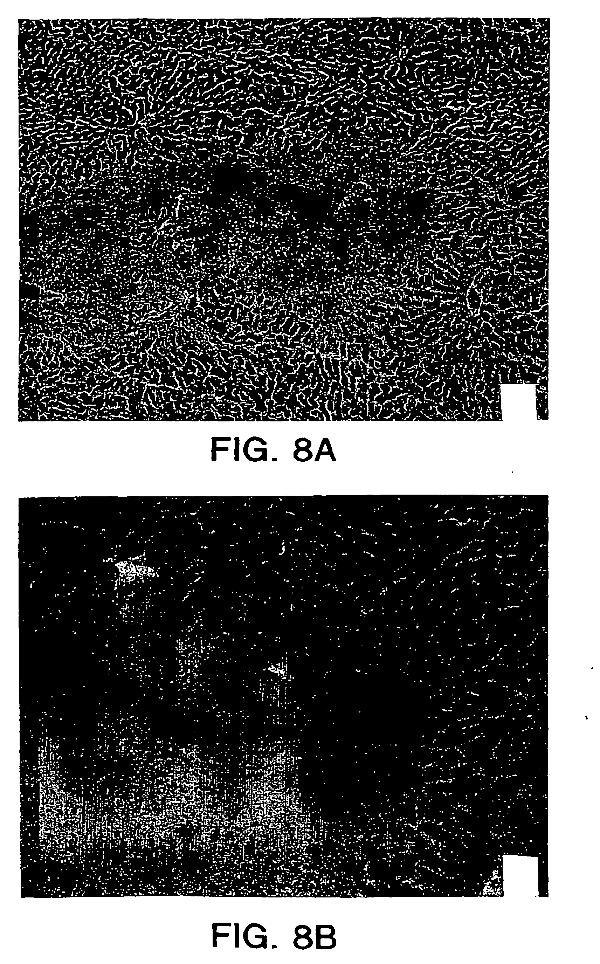

**Figures 8A** and **8B** illustrate the results of MDC Injections into liver tissue. Injections were made into liver tissue In the lower left lobe, and issue samples were prepared 4 days post-injection. MDC are Indicated by β-galactosidase staining. Figure 8A shows low. (100 X) magnification; Figure 3B shows high (200 X) magnification.

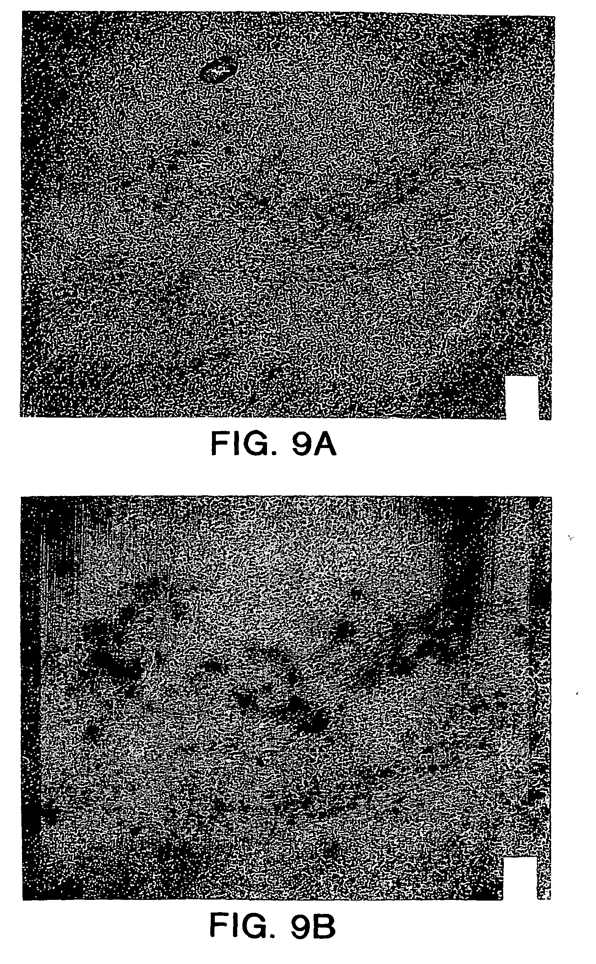

**Figures 9A** and **9B** illustrate the results of MDC Injections into spleen tissue. Injections were made into spleen tissue in the interior aspect, and tissue samples were prepared 4 days post-injection. MDC are indicated by β-galactosidase staining. Figure 9A shows injected tissues viewed by low (100 X) magnification; Figure 9B shows injected tissues viewed by high (200 X) magnification.

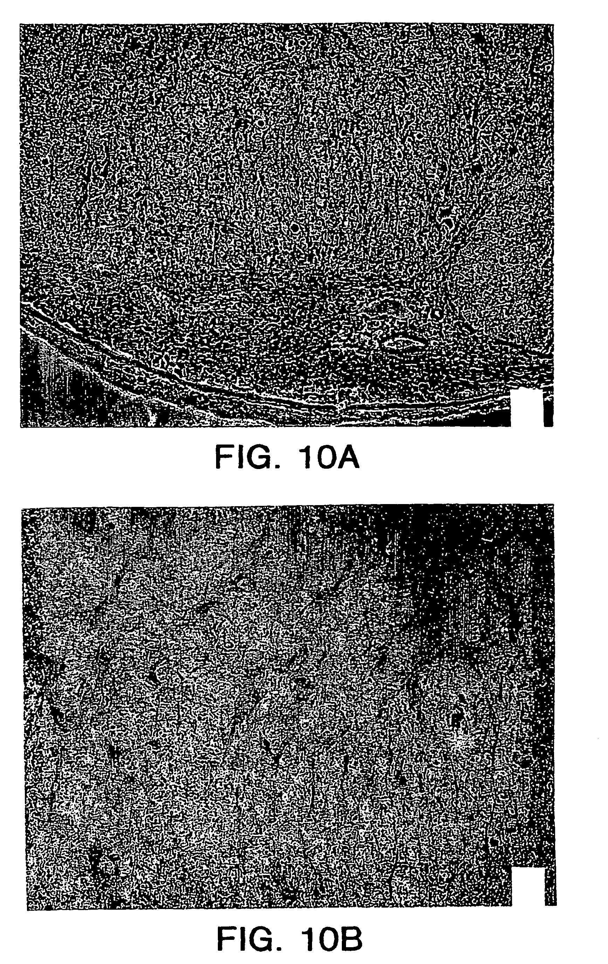

**Figures 10A** and **10B** illustrate the results of MDC injections into spinal cord tissue. Injections were made into spinal cord tissue, and tissue samples were prepared 4 days post-injection. MDC are indicated by β-galactosidase staining. Figure 10A shows Injected tissues viewed by low (100 X) magnification; Figure 10B shows Injected tissues viewed by high (200-X) magnification. Figures 7A-7B, 8A-8B, 9A-9B, and 10A-10B demonstrate that MDC remain viable following injection into a variety of different tissue types without damaging the host tissues.

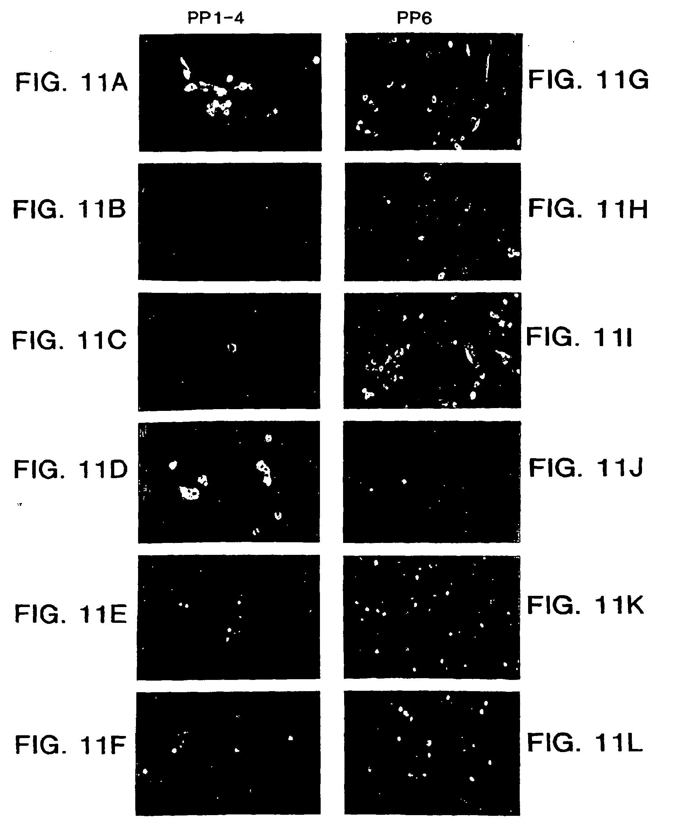

**Figures 11A-11L** illustrate immunohistochemical analysis of PP1-4 and PP6 cell populations from *mdx* mice showing expression of cell markers including desmin, MyoD, and myogenin (markers specific for myogenic lineages), M-cadherin (satellite cell specific marker), Bcl-2 (early myogenesis marker), CD34 (hematopoietic or stromal cell marker). Figures 11A-11L demonstrate that PP1-4 and PP6 cell populations show comparable percentage of cells expressing desmin (Figures 11A and 11G), MyoD (Figures 11 E and 11K), and myogenin (Figures 11F and 11L), while the PP6 population shows a lower percentage of cells expressing M-cadherin (Figures 11D and 11J), but a higher percentage of cells expressing Bcl-2 (Figures 11C and 11I) and CD34 (Figures 11B and 11H), compared with the PP1-4 population.

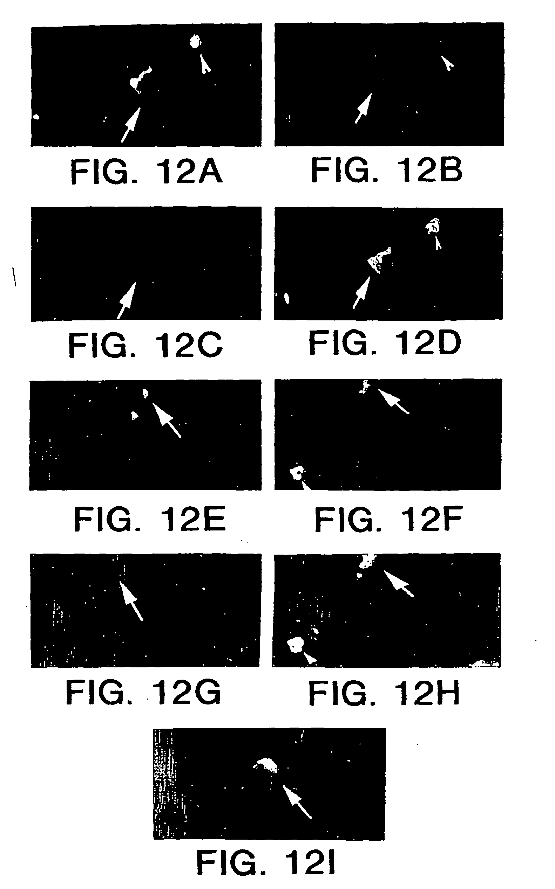

**Figures 12A-12I** illustrate the intracellular co-localization of CD34 or Bcl-2 staining with desmin staining in mouse muscle cells and vascular endothelial cells. Figure 12A shows normal mouse muscle cells (see arrow) and vascular endothelial cells (see arrowhead) stained with anti-CD34 antibodies and visualised by fluorescence microscopy. Figure 12B shows the same cells co-stained with desmin and collagen type IV antibodies. Figure 12C shows the same cells co-stained with Hoechst to show the nuclei. Figure 12D shows a composite of the cells co-stained for CD34, desmin, collagen type IV, and Hoechst. Figure 12E shows normal mouse muscle cells (see arrow) stained with anti-Bcl-2 antibodies and visualized by fluorescence microscopy. Figure 12F shows the same cells co-stained with desmin and collagen type IV antibodies. Figure 12G shows the same cells co-stained with Hoechst to show the nuclei. Figure 12H shows a composite of the cells co-stained for CD34, desmin, collagen type IV, and Hoechst. Figure 12I shows satellite cells stained with anti-M-cadherin antibodies (see arrow). Cells were viewed at 40 X magnification. Figures 12A-12D demonstrate the co-localization of CD34 and desmin, while Figures 12E-12H demonstrate the co-localization of Bcl-2 and desmin.

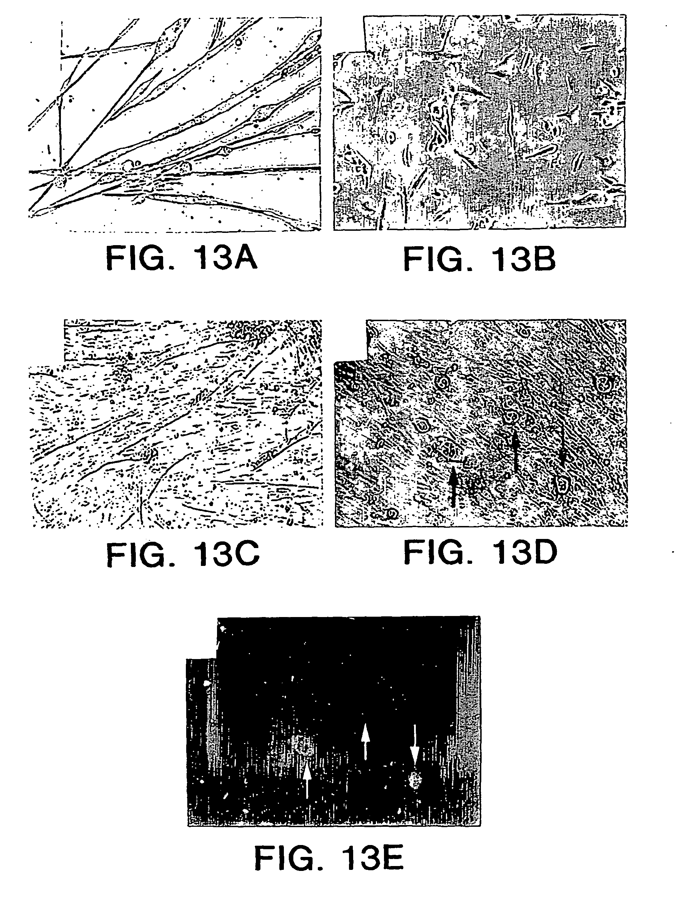

**Figures 13A-13E** illustrate the morphologic changes and expression of osteocalcin resulting from the exposure of mc13 cells to rhBMP-2. Mc13 cells were incubated in growth media with or without rhBMP-2 for 6 days. Figure 13A shows cells grown to >50% cell confluency in the absence of rhBMP-2. Figure 13B shows cells grown to >50% cell confluency in the presence of 200 ng/ml rhBMP-2. Figure 13C shows cells grown to >90% cell confluency in the absence of rhBMP-2. Figure 13D shows cells grown to >90 % confluency in the presence of 200 ng/ml rhBMP-2. Figure 13E shows cells stained for osteocalcin expression (osteoblast cell marker; see arrows). Cells were viewed at 10 X magnification. Figures 13A-13E demonstrate that mc13 cells are capable of differentiating into osteoblasts upon exposure to rhBMP-2.

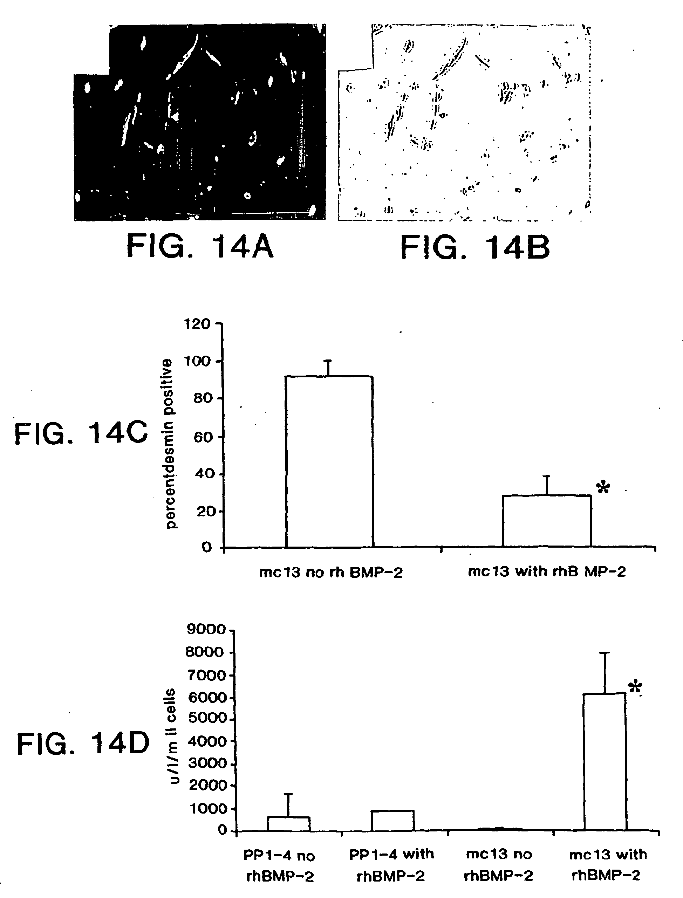

**Figures 14A-14D** illustrate the effects on the percentage of mc13 cells expressing desmin and alkaline phosphatase in response to rhBMP-2 treatment. Figure 14A shows desmin staining of newly isolated mc13 clones. Figure 14B shows a phase contrast view of the same cells. Figure 14C shows the levels of desmin staining in mc13 cells following 6 days of incubation in growth media with or without 200 ng/ml rhBMP-2. Figure 14D shows the levels of alkaline phosphate staining in PP1-4 cells and mc13 cells following 6 days of incubation in growth media with or without 200 ng/ml rhBMP-2.* indicates a statistically significant result (student's t-test). Figure 14C demonstrates that a decreasing number of mc13 cells express desmin in the presence of rhBMP-2, while Figure 14D demonstrates that an increasing number of mc13 cells express alkaline phosphatase in the presence of rhBMP-2, suggesting decreasing myogenic characteristics and increasing osteogenic characteristics of the cells In the presence of rhBMP-2.

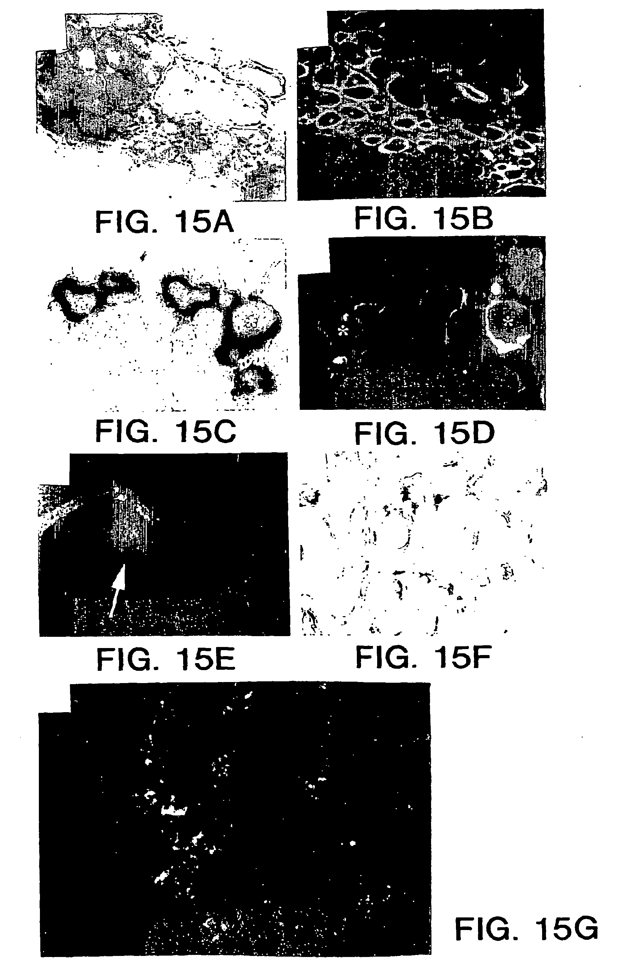

**Figures 15A-15G** illustrate the *in vivo* differentiation of mc13 cells into myogenic and osteogenic lineages. Mc13 cell were stably transfected with a construct containing LacZ and the dystrophin gene, and introduced by intramuscular or intravenous Injection into hind limbs of *mdx* mice. After 15 days, the animals were sacrificed and the hind limb musculature was isolated for histology. Figure 15A shows mc13 cells at the intramuscular injection site stained for LacZ. Figure 15B shows the same cells co-stained for dystrophin. Figure 15C shows mc13 calls in the region of the intravenous injection stained for LacZ. Figure 15D shows the same cells co-stained for dystrophin. In a separate experiment, mc13 cells were transduced with adBMP-2, and 0.5-1.0 x 10⁸ cells were injected into hind limbs of SCID mice. After 14 days, the animals were sacrificed, and the hind limb muscle tissues were analyzed. Figure 15E shows radiographic analysis of the hind limb to determine bone formation. Figure 15F shows the cells derived from the hind limb stained for LacZ. Figure 15G shows cells stained for dystrophin. Figures 15A-15D demonstrate that mc13 cells can rescue dystrophin expression via intramuscular or intravenous delivery. Figures 15E-15G demonstrate that mc13 cells are Involved in ectopic bone formation. Cells were viewed at the following magnifications: 40 X (Figures 15A-15D), 10 X (Figures 15F-15G)

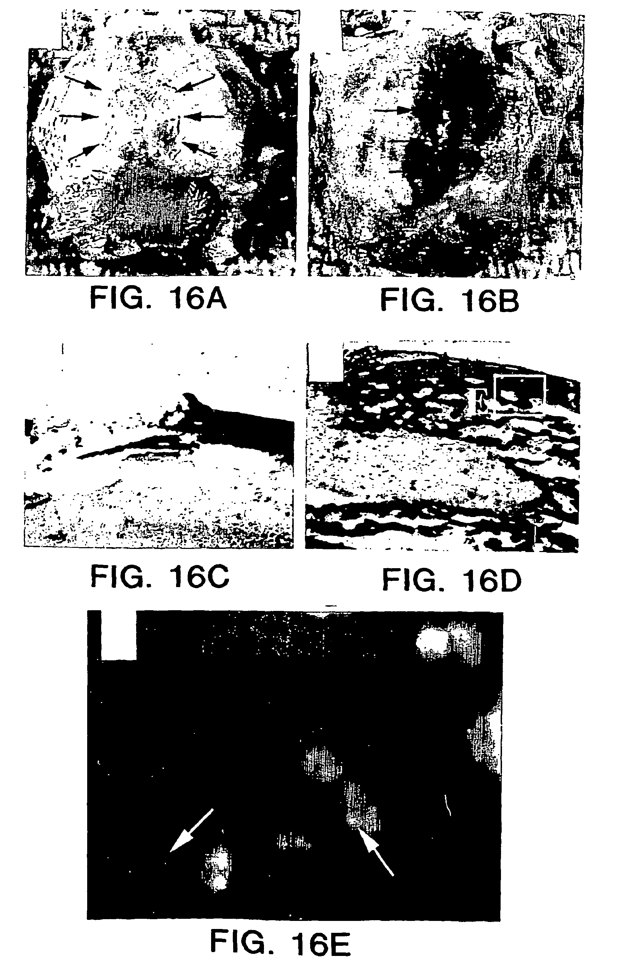

**Figures 16A-16E** illustrate the enhancement of bone healing by rhBMP-2 producing primary muscle cells. A 5 mm skull defect was created in female SCID mice using a dental burr, and the defect was filled with a collagen sponge seeded with mc13 cells with or without adBMP-2. The animals were sacrificed at 14 days, inspected, and analyzed microscopically for indications of bone healing. Figure 16A shows a skull treated with mc13 cells without adBMP-2. Figure 16B shows a skull treated with mc13 cells transduced with adBMP-2. Figure 16C shows a histological sample of the skull treated with mc13 cells without adBMP-2 analyzed by von Kossa staining. Figure 16D shows a histological sample of the skull treated with mc13 cells transduced with adBMP-2 analyzed by von Kossa staining. Figure 16E shows a histological sample of the skull treated with the mc13 cells transduced with adBMP-2 analyzed by hybridization with a Y-chromosome specific probe to identify the injected cells (green fluorescence shown by arrows), and stained with ethidium bromide to identify the nuclei (indicated by red fluorescence). Figures 16A-16E demonstrate that mc13 cells expressing rhBMP-2 can contribute to the healing of bone defects.

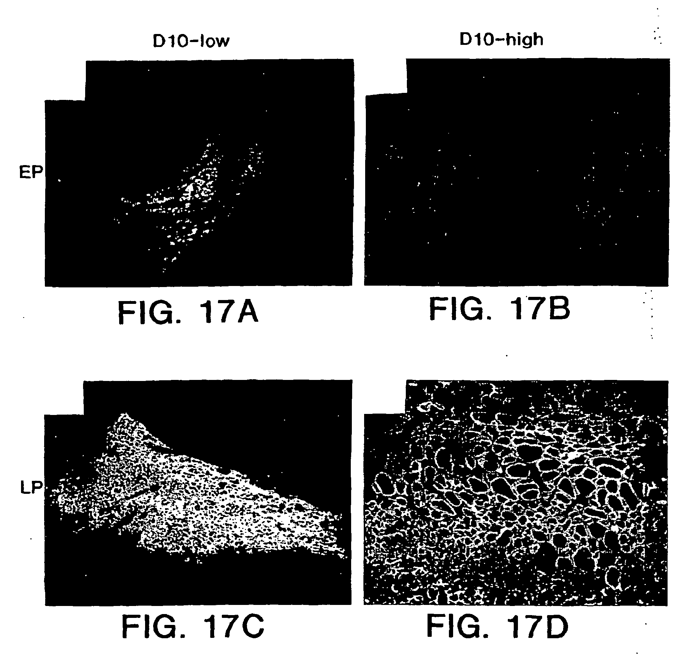

**Figures 17A-17F** show engraftments resulting from the injection of MDSC, either of late preplate (LP), (i.e., MDSC [LP]- PP5 or PP6), or early preplate (EP), (i.e., MDSC [EP])- PP1-2). As shown in Figs. 17C and 17D, a large engraftment with a significant number of dystrophin-positive (dystrophin+) myofibers was observed in the MDSC [LP]-injected mdx muscle by ten days post-injection (Example 10). A comparison of the MDSC [LP]-injected muscle with MDSC [EP]-injected muscle (Figs. 17A and 17B), shows that the MDSC [LP]-engraftment contains many more small myofibers, thus suggesting that the injected MDSC [LP] cells possess a high proliferative ability *in vivo.* In both muscles, the same number of muscle cells of each type was injected. The number of dystrophin+ myofibers in muscle injected with MDSC [LP] was about 5 times that found in muscle injected with MDSC [EP] (2.798 +/- 1.114, n=4 In mdsc vs. 430 +/- 148, n=6 in EP; Mean +/- SD).

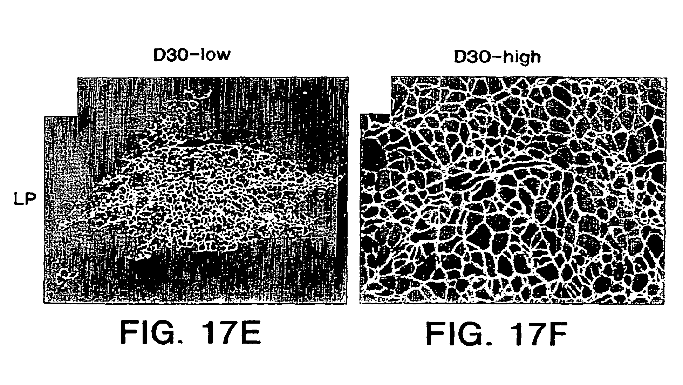

Importantly, when MDSC [LP] were used, many dystrophin+ myofibers were present in muscle by 30 days post-injection (Figs. 17E and 17F). A comparison of Fig. 17C with Fig. 17E indicates that both engraftments had similar areas; however, for the 30-day muscle (Fig. 17E), only half the amount of cells were injected as were injected for the 10-day muscle (Fig. 17C). In addition , the muscle fibers were much larger in the 30-day engraftment than they were in the 10-day engraftment, thus indicating that most of the dystrophin+ myofibers that had formed by 10 days post-injection still survived 20 days later. In contrast, a dramatic decrease in the number of dystrophin+ myofibers was observed in muscle injected with MDSC [EP] by 30 days post-injection, resulting in a greater than ten fold difference in dystrophin+ myofibers between the MDSC [EP] group (134 +/-42, n=3) and the MDSC [LP] group (2,000 +/- 658, n=3).

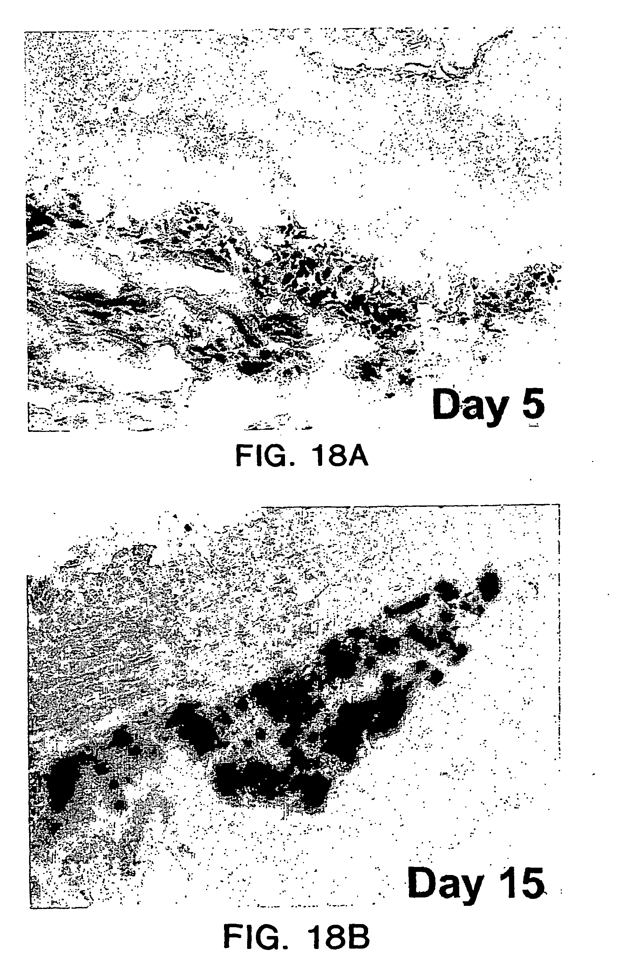

**Figures 18A** and **18B** demonstrate the feasibility of employing human fetal MDSC in the methods according to the present invention. Such cells were found to be immunotolerant and persisted in SCID mice for >2 weeks. 1x10⁸ human fetal MDSC [LP] containing the LacZ gene in an expression vector were injected into the bladder wall of SCID mice. LacZ staining was observed at day 5 (Fig. 18A) and at day 15 (Fig. 18B) post injection, thereby demonstrating excellent survival of the MDSC.

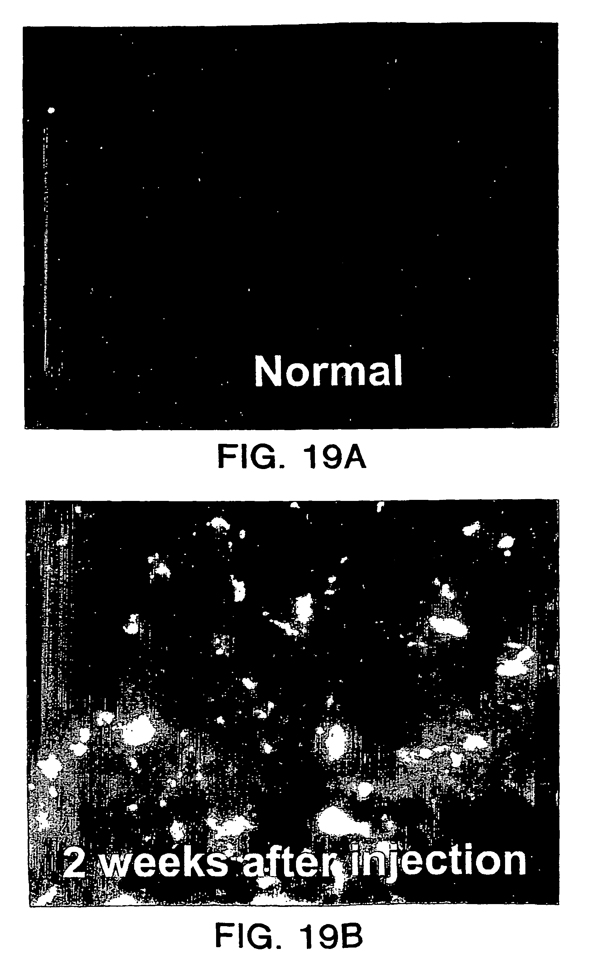

**Figures 19A** and **19B** show desmin staining of normal and MDSC [LP] injected animals. 3x10⁵ MDSC from-normal mice were injected intravenously into non-immunocompromised normal mice. Two weeks later, the thymus was harvested from the injected mice and stained for skeletal muscle specific desmin. Fig. 19A shows that the normal thymus control stains negatively for desmin. However, 2 weeks after peripheral injection of MDSC from another animal, the thymus is positive for desmin staining (Fig. 19B).

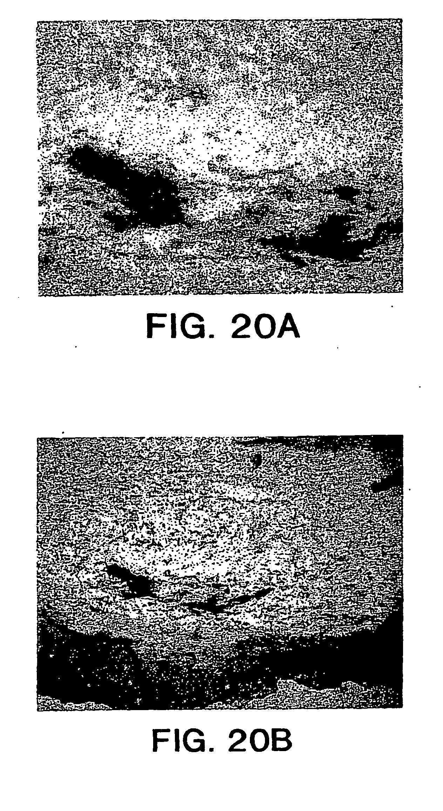

**Figures 20A** and **20B** illustrate the results of injection of MDSC compositions into the spinal disc of rabbits. MDSC contained expression vectors with LacZ for β-galactosidase expression as described. Injections of MDSC obtained from mice were introduced into the spinal disc T6 level of the rabbit At day 10 post-injection, tissue samples were obtained and prepared for analysis. The presence of injected MDSC was indicated by β-galactosidase staining. Figure 20A shows injected tissues at 100 X (high magnification); Figure 20B shows injected tissues at 40 X (low magnification). The results depicted in Figs. 20A and 20B demonstrate that MDC injections persists In the vertebral disc for at least. 10 days and may augment the size and function of normal and dysfunctional discs.

### Muscle-derived cells and compositions

The present invention provides MDC comprised of early progenitor cells (also termed muscle-derived progenitor cells or muscle-derived stem cells (MDSC) herein) that show long-term survival rates following transplantation Into body tissues, preferably soft tissues. To obtain the MDC of this invention, a muscle explant preferably skeletal muscle, is obtained from an animal donor, preferably from a mammal, including humans. This explant serves as a structural and functional syncytium including "rests" of muscle precursor cells (T.A. Partridge et al., 1978, Nature 73:306-8; B.H. Lipton et al., 1979, Science 205:1292-4). The MDC used for transplant or as a cell therapies according to the present invention can be obtained from either autologous or non-autologous (i.e., allogeneic) donors, including human adult, fetal, embryonic, or placental donor cells.

Cells isolated from primary muscle tissue contain a mixture of fibroblasts, myoblasts, adipocytes, hematopoietic, and muscle-derived progenitor cells. The progenitor cells of a muscle-derived population can be enriched using differential adherence characteristics of primary muscle cells on collagen coated tissue flasks, such as described In patent application U.S. Serial No. 09/302,896 of Chancellor et al. Cells that are slow to adhere tend to be morphologically round, express high levels of desmin, and have the ability to fuse and differentiate into multinucleated myotubes (U.S. Serial No. 09/302.896 of Chancellor et al.). A subpopulation of these cells was shown to respond to recombinant human bone morphogenic protein 2 (rhBMP-2) *in vitro* by expressing increased levels of alkaline phosphatase, parathyroid hormone dependent 3', 5'-cAMP, and osteocalcin, Indicative of their ability to differentiate through both osteogenic lineage and myogenic lineages (U.S. Serial No. 09/302,896 of Chancellor et al.; T. Katagiri et al., 1994, J. Cell Biol. 127:1755-1766).

In accordance with the present invention, populations of rapidly adhering MDC (PP1-4) and slowly adhering, round MDC (PP6) were isolated and enriched from skeletal muscle explants and tested for the expression of various markers using immunohistochemistry to determine the presence of pluripotent cells among the slowly adhering cells (Example 1; patent application U.S. Serial No. 09/302,896 of Chancellor et al.). As shown in Table 3, Example 9 herein, the PP6 cells expressed myogenic markers, including desmin, MyoD, and Myogenin. The PP6 cells also expressed c-met and MNF, two genes which are expressed at an early stage of myogenesis (J. B. Miller et al., 1999, Curr. Top. Dev. Biol. 43:191-219; see Table 3). The PP6 showed a lower percentage of cells expressing M-cadherin, a satellite cell-specific marker (A. Irintchev et al., 1994, Development Dynamics 199:326-337), but a higher percentage of cells expressing Bcl-2, a marker limited to cells in the early stages of myogenesis (J. A. Dominov et al., 1998, J. Cell Biol. 142:537-544)*.* The PP6 cells also expressed CD34, a marker identified with human hematopoietic progenitor cells, as well as stromal cell precursors in bone marrow (R. G. Andrews et al., 1986, Blood 67:842-845; C. I. Civin et al., 1984, J. Immunol.133:157-165; L. Fina et al, 1990, Blood 75:2417-2426; P.'J. Simmons et al., 1991, Blood 78:2848-2853; see Table 3).

The PP6 cells also expressed Fik-1, a mouse homologue of human KDR gene which was recently identified as a marker of hematopoietic cells with stem cell-like characteristics (B. L Ziegler et al., 1999, Science 285:1553-1558; see Table 3). Similarly, the PP6 cells expressed Sca-1; a marker present in hematopoietic cells with stem cell-like characteristics (M. van de Rijn et al., 1989, Proc. Natl. Acad. Sci. USA 86:4634-8; M. Osawa et al., 1996, J. Immunol. 156:3207-14; see Table 3). However, the PP6 cells did not express the CD45 or c-Kit hematopoietic stem cell markers (reviewed in LK. Ashman, 1999, lnt. J. Biochem. Cell. Biol. 31:1037-51; GA Koretzky, 1993, FASEB J. 7:420-426; see Table 3).

Preferred in the present invention is the PP6 population of muscle-derived progenitor cells having the characteristics described herein. These muscle-derived progenitor cells express the desmin, CD34, and Bcl-2 cell markers. In accordance with the present invention, the PP6 cells are Isolated by the techniques described herein (Example 1) to obtain a population of muscle-derived progenitor cells that have long-term survivability following transplantation. The PP6 muscle-derived progenitor cell population comprises a significant percentage of cells that express progenitor cell markers such as desmin, CD34, and Bcl-2. In addition, PP6 cells express the Fik-1 and Sca-1 cell markers, but do not express the CD45 or c-Kit markers. Preferably, greater than 95% of the PP6 cells express the desmin, Sca-1, and Flk-1 markers, but do not express the CD45 or c-Kit markers. It is preferred that the PP6 cells are utilized within about 1 day or about 24 hours after the last plating.

As an alternative to the pre-plating method, the MDC of the present invention can be isolated by fluoresoence-activated cell sorting (FACS) analysis using labeled antibodies against one or more of the cell surface markers expressed by the MDC (C. Webster et al., 1988, Exp. Cell. Res. 174:252-65; J.R. Blanton et al., 1999, Muscle Nerve 22:43-50). For example, FACS analysis can be performed using labeled antibodies to directed to CD34, Fik-1, Sca-1, and/or the other cell-surface markers described herein to select a population of PP6-like cells that exhibit long-term survivability when introduced into the host tissue. Also encompassed by the present invention is the use of one or more fluorescence-detection labels, for example, fluorescein or rhodamine, for antibody detection of different cell marker proteins.

### Muscle-derived cell-based treatments

In the present invention, the MDC can be isolated from a skeletal muscle source and introduced or transplanted into a muscle or non-musde soft tissue site of interest, or into bone structures. Advantageously, the MDC of the present invention are isolated and enriched to contain a large number of progenitor cells showing long-term survival following transplantation. In addition, the muscle-derived progenitor celts of this invention express a number of characteristic cell markers, such desmin, CD34, and Bcl-2. Furthermore, the muscle-derived progenitor cells of this invention express the Sca-1, and Flk-1 cell markers, but do not express the CD45 or c-Kit cell markers (see Example 1).

MDC and compositions comprising MDC of the present invention can be used to repair, treat, or ameliorate various aesthetic or functional conditions (e.g. defects) through the augmentation of muscle or non-muscle soft tissues. In particular, such compositions can be used as soft-tissue bulking agents for the treatment of: 1) cosmetic and aesthetic conditions of the skin; 2) conditions of the lumen; 3) gastroesophageal reflux symptoms or conditions; 4) fecal incontinence; 5) skeletal muscle weakness, disease, injury or dysfunction; 6) smooth muscle weakness, disease, injury, or dysfunction; and 7) congenital, degenerative, or traumatic vertebral disc symptoms or conditions, including back pain and deficiency of the disc. In addition, such MDC and compositions thereof can be used for augmenting soft tissue not associated with injury by adding bulk to a soft tissue area, opening, depression, or void in the absence of disease or trauma, such as for "smoothing" or removing a wrinkle. Multiple and successive administrations of MDC are also embraced by the present invention.

For MDC-based treatments, a skeletal muscle explant is preferably obtained from an autologous or heterologous, i.e., allogeneic, human or animal source. An autologous animal or human source is preferred, although allogeneic muscle derived stem cells are highly suitable for use in many instances. MDC compositions are then prepared and isolated as described herein. To introduce or transplant the MDC and/or compositions comprising the MDC according to the present invention into a human or animal recipient, a suspension of mononucleated muscle cells is prepared. Such suspensions contain concentrations of the progenitor cells of the invention in a physiologically-acceptable carrier, excipient, or diluent. For example, suspensions of MDC for administering to a subject can comprise 10⁸ to 10⁹ cells/ml in a sterile solution of complete medium modified to contain the subject's serum, as an alternative to fetal bovine serum. Alternatively, MDC suspensions can be in serum-free, sterile solutions, such as cryopreservation solutions (Celox Laboratories, St. Paul, MN). The MDC suspensions can then be introduced e.g., via injection, Into one or more sites of the donor tissue.

The described cells can be administered as a pharmaceutically or physiologically acceptable preparation or composition containing a physiologically acceptable carrier, excipient, or diluent, and administered to the tissues of the recipient organism of interest, including humans and non-human animals. The MDC-containing composition can be prepared by resuspending the cells In a suitable liquid or solution such as sterile physiological saline or other physiologically acceptable Injectable aqueous liquids. The amounts of the components to be used in such compositions can be routinely determined by those having skill In the art The MDC or compositions thereof can be administered by placement of the MDC suspensions onto absorbent or adherent material, i.e., a collagen sponge matrix, and insertion of the MDC-containing material into or onto the site of interest. Alternatively, the MDC can be administered by parenteral routes of injection, including subcutaneous, intravenous, Intramuscular, and intrasternal. Other modes of administration include, but are not limited to, intranasal, intrathecal, intracutaneous, percutaneous, enteral, and sublingual. In one embodiment of the present invention, administration of the MDC can be mediated by endoscopic surgery.

For injectable administration, the composition Is in sterile solution or suspension or can be resuspended in pharmaceutically- and physiologically-acceptable aqueous or oleaginous vehicles, which may contain preservatives, stabilizers, and material for rendering the solution or suspension isotonic with body fluids (i.e. blood) of the recipient. Non-limiting examples of excipients suitable for use include water, phosphate buffered saline, pH 7.4, 0.15 M aqueous sodium chloride solution, dextrose, glycerol, dilute ethanol, and the like, and mixtures thereof. Illustrative stabilizers are polyethylene glycol, proteins, saccharides, amino acids, inorganic acids, and organic acids, which may be used either on their own or as admixtures. The amounts or quantities, as well as the routes of administration used, are determined on an individual basis, and correspond to the amounts used In similar types of applications or indications known to those of skill in the art

To optimize transplant success, the closest possible immunological match between donor and recipient is desired. If an autologous source is not available, donor and recipient Class I and Class II histocompatibility antigens can Obe analyzed to determine the closest match available. This minimizes or eliminates immune rejection and reduces the need for immunosuppressive or immunomodulatory therapy. If required, immunosuppressive or immunomodulatory therapy can be started before, during, and/or after the transplant procedure. For example, cyclosporin A or other immunosuppressive drugs can be administered to the transplant recipient. Immunological tolerance may also be induced prior to transplantation by alternative methods known in the art (D.J Watt et al., 1984, Clin. Exp. Immunol. 55:419; D. Faustman et al., 1991, Science 252:1701).

Consistent with the present invention, the MDC can be administered to body tissues, including bone, epithelial tissue (i.e., skin, lumen, etc.), connective tissue (i.e., adipose, cartilage, ligament, lymph, etc.), muscle tissue (i.e., skeletal/striated or smooth muscle), and various organ tissues such as those organs that are associated with the digestive system (i.e., mouth, tongue, esophagus, stomach, liver, pancreas, gall bladder, intestine, anus, etc.), cardiovascular system (i.e., heart, veins, arteries, capillaries, etc.), respiratory system (i.e., lungs, trachea, etc.), reproductive system (i.e., vas deferens, scrotum, testes, penis, fallopian tubes, vagina, clitoris, uterus, breasts, ovaries, vulva, etc.), urological system (i.e., bladder, urethra, ureter, kidneys, etc.), and nervous system (i.e., brain, spinal cord, nerves, etc.).

The number of cells in an MDC suspension and the mode of administration may vary depending on the site and condition being treated. As non-limiting examples, in accordance with the present invention, about 1-1.5 x 10⁶ MDC are injected for the treatment of an approximately 8 mm diameter region of cryodamage in bladder smooth muscle tissue (see Example 6), while about 0.5-1.0 x 10⁶ MDC are administered via a collagen sponge matrix for the treatment of an approximately 5 mm region of skull defect (see Example 9). Consistent with the Examples disclosed herein, a skilled practitioner can modulate the amounts and methods of MDC-based treatments according to requirements, limitations, and/or optimizations determined for each case.

### Dermatological conditions

The MDC and compositions thereof according to the present invention have marked utility as materials for soft tissue augmentation in cosmetic procedures, e.g., plastic surgery or anti-aging procedures. Specifically, such MDC and MDC-containing compositions can be used to treat various dermatological conditions in a human or animal subject, Including, but not limited to, wounds, wrinkles, rhytids, cutaneous depressions of non-traumatic origin, stretch marks, depressed scars, scaring from acne vulgaris, and hypoplasia of the lip. More specifically, the MDC and compositions of the present invention can be used to treat wrinkles, rhytids, or cutaneous depressions of the face, and especially, the region surrounding the eye(s). To treat dermatological conditions, the MDC are prepared as disclosed herein and then administered, e.g. via injection, to the skin, subcutaneousty or intradermally, to fill, bulk up, or repair the defect. The number of MDC introduced is modulated to repair deep cutaneous depressions or defects, as well as superficial surface depressions or defects, as required. For example, about 1-1.5 x 10⁶ MDC are utilized for the augmentation of an.approximately 5 mm region of the skin (see Example 3).

### Conditions of the lumen

The MD9 and compositions thereof according to the present invention have further utility as treatments for conditions of the lumen in an animal or mammal subject, Including humans. Specifically, the muscle-derived progenitor cells are used for completely or partially blocking, enhancing, enlarging, sealing, repairing, bulking, or filling various biological lumens or voids within the body. Lumens include, without limitation, blood vessels, intestine, stomach, esophagus, urethra, vagina, Fallopian tubes, vas deferens, and trachea. Voids may include, without limitation, various tissue wounds (i.e., loss of muscle and soft tissue bulk due to trauma; destruction of soft tissue due to penetrating projectiles such as a stab wound or bullet wound; loss of soft tissue from disease or tissue death due to surgical removal of the tissue including loss of breast tissue following a mastectomy for breast cancer or loss of muscle tissue following surgery to treat sarcoma, etc.), lesions, fissures, diverticulae, cysts, fistulae, aneurysms, and other undesirable or unwanted depressions or openings that may exist within the body of an animal or mammal. Including humans. For the treatment of conditions of the lumen, the MDC are prepared as disclosed herein and then administered, e.g. via injection or intravenous delivery, to the lumenal tissue to fill or repair the void. The number of MDC introduced is modulated to repair large or small voids in a soft tissue environment, as required.

### Conditions of the sphincter

The MDC and compositions thereof according to the present invention can also be used for the treatment of a sphincter injury, weakness, disease, or dysfunction In an animal or mammal, including humans. In particular, the MDC are used to augment tissues of the esophageal, anal, cardiac, pyloric, and urinary sphincters. More specifically, the present invention provides soft tissue augmentation treatments for gastroesophageal reflux symptoms, and urinary and fecal incontinence. For the treatment of sphincter defects, the MDC are prepared as described herein and then administered to the sphincter tissue, e.g. via injection, to provide additional bulk, filler; or support. The number of MDC introduced is modulated to provide varying amounts of bulking material as required. For example, about 1-1.5 x 10⁶ MDC are used to provide augmentation for an approximately 5 mm region of the gastroesophageal junction or an approximately 5-10 mm region of the anal sphincter (see Example 4).

### Muscle augmentation and contractility

In the present invention, the MDC and compositions thereof can be used for the treatment of muscle conditions in a human or animal subject In particular, the MDC can be used to augment the skeletal or smooth muscles to treat weakness or dysfunction caused by injury, disease, inactivity, or anoxia- or surgery-induced trauma. More specifically, the present invention provides treatments for skeletal muscle weakness or dysfunction, such as a sports-related injury. The present invention also provides treatments for smooth muscle disease or dysfunction, such as heart failure, or injury associated with myocardial infarction.

For muscle augmentation or treatment of muscle-related conditions, the MDC are prepared as described above and are administered, e.g. via injection, into muscle tissue to provide additional bulk, filler, or support. As is appreciated by the skilled practitioner, the number of MDC introduced is modulated to provide varying amounts of bulking material, as needed or required. For example, about 1-1.5 x 10⁸ MDC are injected for the augmentation of an approximately 5 mm region of heart tissue (see Example 7).

In addition, the MDC and compositions thereof can be used to affect contractility in smooth muscle tissue, such as gastrointestinal tissue, esophageal tissue, and bladder tissue, as example. Indeed, muscle contractility was seen to be restored in cryodamaged bladder tissue after the introduction of muscle-derived progenitor cells, i.e., MDC, as demonstrated in Example 6. Thus, the present invention also embraces the use of MDC of the Invention in restoring muscle contraction, and/or ameliorating or overcoming smooth muscle contractility problems, such decreased gastrointestinal motility, including the esophagus, stomach and Intestine smooth muscle. A specific, yet nonlimiting example of a condition that the MDC of the invention can improve, reduce, or correct Is gastroparesis, i.e., poor motility and emptying of the stomach.

### Genetically engineered muscle-derived cells

The MDC of this invention may be genetically engineered to contain a nucleic acid sequence(s) encoding one or more active biomolecules, and to express these biomolecules, including proteins, polypeptides, peptides, hormones, metabolites, drugs, enzymes, and the like. Such MDC may be histocompatible (autologous) or nonhistocompatible (allogeneic) to the recipient, including humans. These cells can serve as long-term local delivery systems for a variety of treatments, for example, for the treatment of such diseases and pathologies as cancer, transplant rejection, and the regeneration of muscle and nerve tissues, diabetes, liver failure, renal failure, neural defects and diseases such as Parkinson's disease, and to deliver a gene product to a site of tissue augmentation, or void filling, such as a therapeutic agent, as described herein.

Preferred in the present invention are autologous muscle-derived progenitor cells, which will not be recognized as foreign to the recipient. In this regard, the MDC used for cell-mediated gene transfer or delivery will desirably be matched vis-à-vis the major histocompatibility locus (MHC or HLA in humans). Such MHC or HLA matched cells may be autologous. Alternatively, the cells may be from a person having the same or a similar MHC or HLA antigen profile. The patient may also be tolerized to the allogeneic MHC antigens. The present invention also encompasses the use of cells lacking MHC Class I and/or II antigens, such as described in U.S. Patent No. 5,538,722.

The MDC may be genetically engineered by a variety of molecular techniques and methods known to those having skill in the art, for example, transfection, infection, or transduction. Transduction as used herein commonly refers to cells that have been genetically engineered to contain a foreign or heterologous gene via the introduction of a viral or non-viral vector into the cells. Transfection more commonly refers to cells that have been genetically engineered to contain a foreign gene harbored in a plasmid, or non-viral vector. MDC can be transfected or transduced by different vectors and thus can serve as gene delivery vehicles to transfer the expressed products into muscle.

Although viral vectors are preferred, those having skill in the art will appreciate that the genetic engineering of cells to contain nucleic acid sequences encoding desired proteins or polypeptides, cytokines, and the like, may be carried out by methods known in the art, for example, as described in U.S. Patent No. 5,538,722, including fusion, transfection, lipofection mediated by the use of liposomes, electroporation, precipitation with DEAE-Dextran or calcium phosphate, particle bombardment (biolistics) with nucleic acid-coated particles (e.g., gold particles), microinjection, and the like.

Vectors for introducing heterologous (i.e., foreign) nucleic add (DNA or RNA) into muscle cells for the expression of bioactive products are well known in the art. Such vectors possess a promoter sequence, preferably, a promoter that is cell-specific and placed upstream of the sequence to be expressed. The vectors may also contain, optionally, one or more expressible marker genes for expression as an indication of successful transfection and expression of the nucleic acid sequences contained in the vector.

Illustrative examples of vehicles or vector constructs for transfection or infection of the muscle-derived cells of the present invention include replication-defective viral vectors, DNA virus or RNA virus (retrovirus) vectors, such as adenovirus, herpes simplex virus and adeno-associated viral vectors. Adeno-associated virus vectors are single stranded and allow the efficient delivery of multiple copies of nucleic acid to the cell's nucleus. Preferred are adenovirus vectors. The vectors will normally be substantially free of any prokaryotic DNA and may comprise a number of different functional nucleic acid sequences. Examples of such functional sequences include polynucleotide, e.g., DNA or RNA, sequences comprising transcriptional and translational initiation and termination regulatory sequences, Including promoters (e.g., strong promoters, inducible promoters, and the like) and enhancers which are active in muscle cells.

Also included as part of the functional sequences is an open reading frame (polynucleotide sequence) encoding a protein of interest; flanking sequences may also be included for site-directed integration. In some situations, the 5'-flanking sequence will allow homologous recombination, thus changing the nature of the transcriptional initiation region, so as to provide for inducible or noninducible transcription to increase or decrease the level of transcription, as an example.

In general, the nucleic acid sequence desired to be expressed by the muscle-derived progenitor cell is that of a structural gene, or a functional fragment, segment or portion of the gene, that is heterologous to the muscle-derived progenitor cell and encodes a desired protein or polypeptide product, for example. The encoded and expressed product may be intracellular, i.e., retained in the cytoplasm, nucleus, or an organelle of a cell, or may be secreted by the cell. For secretion, the natural signal sequence present in the structural gene may be retained, or a signal sequence that is not naturally present in the structural gene may be used. When the polypeptide or peptide is a fragment of a protein that is larger, a signal sequence may be provided so that, upon secretion and processing at the processing site, the desired protein will have the natural sequence. Examples of genes of interest for use in accordance with the present invention include genes encoding cell growth factors, cell differentiation factors, cell signaling factors and programmed cell death factors. Specific examples include, but are not limited to, genes encoding BMP-2 (rhBMP-2), IL-1 Ra, Factor IX. and connexin 43.

As mentioned above, a marker may be present for selection of cells containing the vector construct. The marker may be an Inducible or non-Inducible gene and will generally allow for positive selection under induction, or without induction, respectively. Examples of commonly-used marker genes include neomycin, dihydrofolate reductase, glutamine synthetase, and the like.

The vector employed will generally also include an origin of replication and other genes that are necessary for replication in the host cells, as routinely employed by those having skill in the art. As an example, the replication system comprising the origin of replication and any proteins associated with replication encoded by a particular virus may be included as part of the construct. The replication system must be selected so that the genes encoding products necessary for replication do not ultimately transform the muscle-derived cells. Such replication systems are represented by replication-defective adenovirus constructed as described, for example, by G. Acsadi et al., 1994, Hum. Mol. Genet 3:579-584, and by Epstein-Barr virus. Examples of replication defective vectors, particularly, retroviral vectors that are replication defective, are BAG; described by Price et al., 1987, Proc. Natl. Acad. Sci. USA, 84:156; and Sanes et al., 1986, EMBO J., 5:3133. It will be understood that the final gene construct may contain one or more genes of interest, for example, a gene encoding a bioactive metabolic molecule. In addition, cDNA, synthetically produced DNA or chromosomal DNA may be employed utilizing methods and protocols known and practiced by those having skill in the art

If desired, infectious replication-defective viral vectors may be used to genetically engineer the cells prior to *in vivo* injection of the cells. In this regard, the vectors may be introduced into retroviral producer cells for amphotrophic packaging. The natural expansion of muscle-derived progenitor cells into adJacent regions obviates a large number of injections into or at the site(s) of interest.

The present invention also provides *ex vivo* gene delivery to cells and tissues of a recipient mammalian host, including humans, through the use of MDC, e.g., early progenitor muscle cells, that have been virally transduced using an adenoviral vector engineered to contain a heterologous gene encoding a desired gene product. Such an *ex vivo* approach provides the advantage of efficient viral gene transfer, which is superior to direct gene transfer approaches. The *ex vivo* procedure involves the use of the muscle-derived progenitor cells from isolated cells of muscle tissue. The muscle biopsy that will serve as the source of muscle-derived progenitor cells can be obtained from an injury site or from another area that may be more easily obtainable from the clinical surgeon.

It will be appreciated that in accordance with the present invention, clonal isolates can be derived from the population of muscle-derived progenitor cells (i.e., PP6 cells) using various procedures known in the art, for example, limiting dilution plating in tissue culture medium. Clonal isolates comprise genetically identical cells that originate from a single, solitary cell. In addition, clonal isolates can be derived using FACS analysis as described above, followed by limiting dilution to achieve a single cell per well to establish a clonally isolated cell line. An example of a clonal isolate derived from the PP6 cell population is mc13, which is described in Example 9. Preferably, MDC clonal isolates are utilized in the present methods, as well as for genetic engineering for the expression of one or more bioactive molecules, or in gene replacement therapies.

The MDC are first infected with engineered viral vectors containing at least one heterologous gene encoding a desired gene product, suspended in a physiologically acceptable carrier or excipient, such as saline or phosphate buffered saline, and then administered to an appropriate site in the host. Consistent with the present invention, the MDC can be administered to body tissues, including bone, epithelial tissue, connective tissue, muscle tissue, and various organ tissues such as those organs that are associated with the digestive system, cardiovascular system, respiratory system, reproductive system, urological system, and nervous system, as described above. The desired gene product is expressed by the injected cells, which thus introduce the gene product into the host. The introduced and expressed gene products can thereby be utilized to treat, repair, or ameliorate the injury, dysfunction, or disease, due to their being expressed over long time periods by the MDC of the invention, having long-term survival in the host.

In animal model studies of myoblast-mediated gene therapy, implantation of 10⁶ myoblasts per 100 mg muscle was required for partial correction of muscle enzyme defects (see, J.E. Morgan et al., 1988, J. Neural. Sci. 86:137; T.A. Partridge et al., 1989, Nature 337:176). Extrapolating from this data, approximately 10¹² MDC suspended in a physiologically compatible medium can be implanted into muscle tissue for gene therapy for a 70 kg human. This number of MDC of the invention can be produced from a single 100 mg skeletal muscle biopsy from a human source (see below). For the treatment of a specific injury site, an injection of genetically engineered MDC into a given tissue or site of injury comprises a therapeutically effective amount of cells in solution or suspension, preferably, about 10⁵ to 10⁸ cells per cm³ of tissue to be treated, In a physiologically acceptable medium.

### Allogeneic muscle-derived stem cells provide effective cell transplantation

Efficient cell transplantation has been obtained by injecting the MDSC of the late preplates, e.g., PP5-6, as described, into a non-autologous (allogeneic) host. For example, large numbers of cells comprising allogeneic grafts were observed In the hosts' muscles at 30 days post-injection (e.g., >2000 dystrophin+ myofibers were found in host muscles after injection of 3x10⁵ muscle-derived stem cells of non-host origin). (Example 10). This result indicates that the injected MDSC not only circumvented the generally poor spread and poor survival that follow injection, but also that the injected cells bypassed immuno-rejection In host muscles, which is often observed In cell transplantation between different transplant donor MDSC having different origin from the host, e.g., as in different strains of mice. The results demonstrated that non-autologous muscle derived stem cells significantly improved the efficiency of cell therapy in diseased muscles of the host In addition, MDSC can be considered to be immunoprivileged. Allogeneic MDSC cells of the late preplating (e.g., PP5 or 6) survive more than 10 times longer than nonstem cells from early prelates, e.g., PP1-2 or PP1-4, when injected or transplanted Into a host animal of a different strain.

Table 1 presents the results described in Example 10 and Figures 17A-17F comparing the use of normal MDSC [EP] and MDSC [LP] cells transplanted into mdx host mice. The data in Table 1 represent the number of dystrophin-positive (dystrophin+) myofibers found in mdx muscle injected with either MDSC [EP] or MDSC [LP] stem cells.

**TABLE 1**

| | MDSC [EP] | | MDSC [LP] | |
|---|---|---|---|---|
| | M | SD | M | SD |
| Day 10 | 430.7 | 147.9 | 2798.0 | 1141.6 |
| Day 30 | 134.0 | 41.6 | 2000.1 | 657.6 |

| | | | | |
|---|---|---|---|---|
| 3-6 muscles per group; M = mean; SD = standard deviation | | | | |

The immunoprivileged nature of the MDSC of the late-preplate cells is supported by immunohistochemical results (Desmin staining) which show that when MDSC are injected peripherally, they can and do migrate to the thymus. Thereafter the injected MDSC may differentiate into T-lymphocytes and induce chimeric tolerance. (e.g., Fig.19 - positive Desmin thymus staining after peripheral MDSC injection).

In addition, compared with MDSC [EP], some MDSC [LP] can differentiate not only into mature cells of muscle, or different lineage, following injection into a host animal, but also into satellite cells when injected into host muscle, in such cases, a population of MDSC [LP] cells localize in the muscle, as well as in the basal lamina of myofibers which is a site of satellite cells. Cells derived from the MDSC [LP] and localized in the basal lamina of myofibers become M-cadherin-positive over time. Newly-created satellite cells in these sites can form new myofibers, for example, If the host myofibers die. Without wishing to be bound by theory, the MDSC [LP] which migrate to the basal lamina of myofibers may respond to signals or factors at and around this site which cause them to develop Into satellite cells. Therefore, MDSC [LP] injection affords a means for providing a future sustained population of muscle precursor cells which form satellite cells at host muscle sites where satellite cells exist and develop.

The invention also provides the ability to employ human fetal or embryonic MDSC in transplant methodologies and treatments, under appropriate guidelines and approved conditions and regulations, with minimal to no problems of rejection due to donor-host incompatibilities. For example, human MDSC from fetal limb muscle were found to be immunotolerant and to exhibit high levels of survivability, as they were able to persist in SCID mice for >2 weeks (Figs. 18A and 18B) post injection. Thus, in accordance with the invention and under the appropriate guidelines, regulations and conditions, banked human fetal MDSC, for example, may be utilized and injected into any patients tissues or organs for treatments that are amenable to MDSC injection or transplant as described herein.

### EXAMPLE 1: MDC enrichment, isolation and analysis

### Enrichment and isolation of MDC

MDC were prepared as described (patent application U.S. Serial No. 09/302,896 of Chancellor et al.). Muscle explants were obtained from the hind limbs of a number of sources, namely from 3-week-old *mdx* (dystrophic) mice (C57BL/10ScSn *mdx*/*mdx,* Jackson Laboratories), 4-6 week-old normal female SD (Sprague Dawley) rats, or SCID (severe combined immune deficiency) mice. The muscle tissue from each of the animal sources was dissected to remove any bones and minced into a slurry. The slurry was then digested by 1 hour serial incubations with 0.2% type XI collogenase, dispase (grade II, 240 unit), and 0.1 % trypsin at 37°C. The resulting cell suspension was passed through 18,20, and 22 gauge needles and centrifuged at 3000 rpm for 5 minutes. Subsequently, cells were suspended in growth medium (DMEM supplemented with 10% fetal bovine serum, 10% horse serum, 0.5% chick embryo extract, and 2% penicillin/streptomycin). Cells were then preplated in collagen-coated flasks (patent application U.S. Serial No. 09/302,896 of Chancellor et al.). After approximately 1 hour, the supernatant was removed from the flask and replated into a fresh collagen-coated flask. The cells which adhered rapidly within this 1 hour incubation were mostly fibroblasts (Z. Qu et al., *supra;* application U.S. Serial No. 09/302,896 of Chancellor et al.). The supernatant was removed and re-ptated after 30-40% of the cells had adhered to each flask. After approximately 5-6 serial platings, the culture was enriched with small, round cells, designated as PP6 cells, which were isolated from the starting cell population and used in further studies. The adherent cells isolated In the early platings were pooled together and designated as PP1-4 cells.

The *mdx* PP1-4, *mdx* PP6, normal PP6, and fibroblast cell populations were examined by immunohistochemical analysis for the expression of cell markers. The results of this analysis are shown in Table 2.

**Table 2**

| | ***mdx* PP1-4 cells** | ***Mdx* PP6 cells** | **nor PP6 cells** | **fibroblasts** |
|---|---|---|---|---|
| **desmin** | +/- | + | + | - |
| **CD34** | - | + | + | - |
| **Bcl-2** | (-) | + | + | - |
| **Flk-1** | na | + | + | - |
| **Sca-1** | na | + | + | **-** |
| **M-cadherin** | -/+ | -/+ | -/+ | - |
| **MyoD** | -/+ | +/- | +/- | - |
| **myogenin** | -/+ | +/- | +/- | - |

| | | | | |
|---|---|---|---|---|
| Table 2: Cell markers expressed in PP1-4 and PP6 cell populations. *Mdx* PP1-4, *mdx* PP6, normal PP6, and fibroblast cells were derived by preplating technique and examined by immunohistochemical analysis. "-" indicates less than 2% of the cells showed expression; "(-)"; "-/+" indicates 5-50% of the cells showed expression; "+/-" indicates -40-80% of the cells showed expression; "+" indicates that > 95% of the cells showed expression; "nor" indicates normal cells; "na" indicates that the immunohistochemical data is not available. | | | | |

It is noted that both *mdx* and normal mice showed identical distribution of all of the cell markers tested in this assay. Thus, the presence of the *mdx* mutation does not affect the cell marker expression of the isolated PP6 muscle-cell derived population.

MDC were grown in proliferation medium containing DMEM (Dulbecco's Modified Eagle Medium) with 10% FBS (fetal bovine serum), 10% HS (horse serum), 0.5% chick embryo extract, and 1% penicillin/streptomycin, or fusion medium containing DMEM supplemented with 2% fetal bovine serum and 1 % antibiotic solution. All media supplies were purchased through Gibco Laboratories (Grand Island, NY).

### EXAMPLE 2: MDC vectors and transfection

### Retrovirus and adenovirus vectors

The MFG-NB (N. Ferry et al., 1991, Proc. Natl. Acad. Sci. USA 88:8377-81) retroviral vector was used for the MDC experiments. This vector contains a modified LacZ gene (NLS-LacZ) that includes a nuclear-localization sequence cloned from the simian virus (SV40) large T antigen transcribed from the long terminal repeat (LTR). The retroviral stock was grown and prepared as previously described (J.C. van Deutekom et al., 1998, Neuromuscul. Disord. 8:135-48). The retrovirus was titered to 1 x 10⁷ to 1 x 10⁹ cfu/ml.

An adenovirus vector was also used. This vector contained the LacZ gene under the control of the human cytomegalovirus (HuCMV) promoter (J. Huard et al., 1994, Hum Gene Ther 5:949-58)*.* The E1-E3 deleted recombinant adenovirus was obtained through Dr. 1. Kovesdi (Gene Vec Inc., Rockville, MD).

### Viral transduction of MDC

For viral transduction, MDC were plated at a density of 1-1.5 x 10⁶ In T 75 flasks. PP6 MDC were washed In HBSS (Hank's Balanced Salt Solution) and Incubated with either retrovirus (1 x 10⁷-1 x 10⁹ cfu/ml) or adenovirus (1 x 10⁹ cfu/ml) suspensions In 5 ml of DMEM containing 8 µg/ml Polybrene^{™} (Abbott Laboratories, Chicago, IL) for 4 h at 37°C. Virally transduced MDC were grown in 10 ml of proliferation medium for 24 h at

### 37°C. MDC were then rinsed with HBSS and enzymatically digested with

0.25% trypsin for 1 minute. The treated, virally transduced MDC were centrifuged for 5 minutes at 3,500 rpm, and the pellet was resuspended In 20 µl of HBSS.

### EXAMPLE 3: Soft tissue augmentation of the skin

### MDC and collagen injection

SD rats were prepared for surgery by anesthetizing with halothane using standard methods, and washing the surgical site with Betadine® solution. The skin of the lower abdomen was injected with either 10 microliters (µl) of a MDC suspension in HBSS (approximately 1-1.5 x 10⁸ cells), 10 µl of commercially available bovine collagen (Contigen^{™}; C.R. Bard, Covington, GA), or 10 µl of sterile saline using a Hamilton microsyringe. At 5 days, 2 weeks and 4 weeks post-injection, the area surrounding each injection site was excised, prepared for histochemical analysis, examined microscopically, and photographed. Histochemical analysis included hematoxylin, eosin, or trichrome staining.

The results demonstrate that MDC were viable for up to at least 4 weeks following injection into skin tissue, with no evidence of inflammation of the tissue at the injection site (Figures 1D-1F). In contrast, collagen was not visible at 2 weeks following injection into skin tissue (Figures 1B and 1C). Thus, MDC compositions can be used as skin augmentation materials for use, for example, in cosmetic and aesthetic applications or surgery. This is an unexpected finding, since it was previously believed that transplanted muscle cells needed surrounding host muscle fibers with which to attach in order to survive. The survival of the MDC of the present invention following injection into non-muscle tissue is further demonstrated in Examples 8 and 9.

### EXAMPLE 4: Soft tissue augmentation of the gastroesophageal junction and anal sphincter

SD rats were prepared for surgery as described above. A midline abdomen incision was made to expose the gastroesophageal junction and anal sphincter. The soft tissue was injected with 10 µl of a suspension of muscle-derived progenitor cells of in HBSS (1-1.5 x 10⁶ cells) using a Hamilton microsyringe. At day 3 post-injection, the area surrounding each injection site was excised, prepared for histochemical analysis, stained for β-galactosidase to determine the location and viability of the cells carrying the LacZ marker, examined microscopically, and photographed. Results of these experiments demonstrate that MDC compositions can be used as esophageal and anal sphincter bulking materials (Figures 2A and 2B) for the treatment of gastroesophageal reflux or fecal incontinence symptoms or conditions.

### EXAMPLE 5: Soft tissue augmentation of the vesico-ureteral junction

SD rats were prepared for surgery as described above. A midline abdomen incision was made to expose the ureteral-bladder (vesico-ureteral) junction. The tissue was injected with 10 µl of MDC suspension in HBSS (1-1.5 x 10⁶ cells) using a Hamilton microsyringe. At 3 days post-injection, the area surrounding each injection site was excised, prepared for histological analysis, stained for β-galactosidase to determine the location and viability of the cells carrying the LacZ marker, examined microscopically, and photographed. These results demonstrate that MDC-based compositions can be used as utereral-bladder augmentation materials (Figures 3A and 3B) for the treatment of vesico-utereal reflux symptoms or conditions.

### EXAMPLE 6: MDC treatment of cryodamaged bladder tissue

### Cryoinjury and MDC transplantation

SD rats were prepared for surgery as described above. A low midline incision was made to expose the bladder and urethra. The bladder was then filled with 1 ml saline. Cryodamage was performed with an 8 mm diameter aluminum rod chilled on dry ice. The chilled probe was placed against one side of the bladder wall for 15 or 30 seconds (referred to as "mild" or "severe" damage, respectively), Immediately following cryoinjury, one severe damage group was injected with muscle-derived cells of the invention (1-1.5 x 10⁶ of cells in 15 µl HBSS), while a control severe damage group was injected with HBSS (15 µl) (n = 3 per group). One week following cryoinjury, the other mild and severe damage groups were injected with an MDC suspension in 50 µl HBSS (2-3 x 10⁶ cells), while control mild and severe damage groups were injected with 50 µl HBSS (n = 4 per group). For each group, injections were made into the center of the injured region using a 30-gauge needle and a Hamilton microsyringe.

### Immunohistochemical staining for smooth muscle actin (α-SM actin)

To prepare samples for immunohistochemical analysis, tissues or cell samples were fixed in cold acetone at -20°C for 2 minutes, and blocked with 5 % HS for 1 hour. The samples were incubated overnight at room temperature in a humidity chamber with mouse monoclonal anti-smooth muscle actin primary antibodies (Cat # F-3777; Sigma Chemical Co., St. Louis, MO) (1:400 dilution in PBS pH 7.4). The samples were then washed 3 times with PBS, and incubated with anti-mouse IgG secondary antibodies conjugated with the Cy3 fluorochrome (Sigma Chemical Co.) (1:200 dilution In PBS pH 7.4).

### Immunohistochemical staining for fast myosin heavy chain (Fast MyHC)

Tissues or cell samples were fixed in cold acetone at -20°C for 2 minutes and blocked with 5 % HS for 1 hour. The samples were then incubated overnight at room temperature in a humidity chamber with mouse monoclonal anti-skeletal myosin (fast) primary antibodies. (Cat. # M-4276; Sigma Chemical Co.) (1:400 dilution in PBS pH 7.4). The samples were then washed 3 times with PBS, and incubated with Cy3 conjugated anti-mouse IgG secondary antibodies (Sigma Chemical Co.) (1:200 dilution In PBS pH 7.4).

### Cell culture

Muscle derived progenitor cells as prepared in Example 1 were plated in 35 mm collagen-coated dishes in proliferation medium. After 24 hours, the proliferation medium was replaced with fusion medium. The cells were maintained in fusion medium with daily medium changes until the MDC differentiated into myotubes.

### Contractility studies

Two weeks after the MDC injection, the animals were euthanized and used to prepare bladder strips. Two strips were prepared from each bladder, and both strips were cut to extend along the circumference of the bladder. The bladder strips were mounted in a tissue bath and subjected to neural contractions (20 Hz, 10 and 80 shocks), which were recorded, and analyzed as described below.

### Tissue harvest and histology

SD rats were euthanized and samples of the tissue surrounding the injection site were removed. The samples were flash frozen using 2-methylbutane pre-cooled in liquid nitrogen. Histochemical analysis of the samples included hematoxylin and eosin staining. The samples were stained, examined microscopically, and photographed. Each cryostat section measured 10 µm in thickness.

### Electrostimulation of bladder smooth muscle tissue

The animal was euthanized and the bladder was quickly removed. Two strips covering the circumference of the bladder wall were obtained from each bladder. The strips were mounted in 5 ml organ baths containing Kreb's solution (113 mmol/l NaCl, 4.7 mmol/l KCl, 1.25 mmol/l CaCl₂, 1.2 mmol/l MgSO₄, 25 mmol/l NaHCO₃, 1.2 mmol/l KH₂PO₄, and 11.5 mmol/l glucose) aerated with 95% O₂ and 5% CO₂. The initial tension was set to 10 mN, and isometric contractions were measured with strain-gauge transducers coupled with a TBM4 strain gauge amplifier (World Precision instruments). Contraction measurements were compiled using a data acquisition program (Windaq, DATAQ Instruments, Inc., Akron, OH). The sampling rate per channel was set to 100 Hz. The amplitude of the contractions was computed using a calculation program (WindaqEx, DATAQ Instruments, inc.). Following a 20 minutes equilibration period, electrical field stimuli were applied through two platinum wire electrodes separated by 4 cm at the top and the bottom of the organ bath. The temperature was maintained at 37°C throughout the experiment.

### Chemical stimulation of bladder smooth muscle tissue

The bladder strips were stimulated with square wave pulses of 0.25 msec duration with maximal voltage (100 V) and a frequency response curve constructed using 10 or 80 shocks at 1,2,5,10,20, or 40 Hz Following electrostimulation, 5,10, or 20 µM carbachol was added to the bladder strips to induce contractions, in parallel experiments, 1 µM atropine was added, electrostimulation was applied as above, and 50 µM methylene ATP was added to induce contractions.

### Staining for Innervation

Acetylcholine (Ach) staining was used to assess the reinnervation of MDC in smooth muscle. Ach is a stain for the neuromuscular junction that indicates the presence of nerve endings. Following MDC injection, tissue was excised at day 3, 15, 30, or after 6 months, stained for Ach, observed by microscopy, and photographed.

### Statistical analysis

Values are reported as means ± standard deviations. A "P" value of less than 0.05 was considered statistically significant. Student's test was used.

### MDC differentiation

Muscle derived progenitor cells as prepared in Example 1 were evaluated for cellular differentiation. Alpha-SM actin is the earliest known marker for the smooth muscle cell phenotype (K.M. McHugh, 1995, Dev. Dyn. 204:278-90), and the main marker of the myofibroblastic phenotype (I. Darby et al., 1990, Lab. Invest. 63:21-9). During muscle cell differentiation, expression of α-SM actin decreases, while fast MyHC expression increases. Histochemical analysis of MDC-treated bladder tissues utilizing α-SM actin and fast MyHC markers demonstrates the differentiation of MDC following injection into site of cryoinjury. At day 5 following injection into cryodamaged bladder tissue, several MDC (at least 20%) show α-SM actin staining (Figure 5B), indicating that the cells are still undifferentiated. After 6 months following injection, however, virtually all MDC have differentiated into myotubes or myofibers, as shown by an decrease In α-SM actin staining (Figure 5F), with a concomitant increase in fast MyHC staining (Figure 51).

### Muscle reinnervation

Because acetylcholine (Ach) is present at the neuromuscular junction, it can serve as an indicator of muscle innervation. Histochemical analysis of MDC-treated bladder tissues utilizing the Ach marker demonstrates the reinnervation of the MDC following injection into sites of cryodamage. At day 3 following injection into cryodamaged bladder tissue the Injected MDC show minimal innervation, as indicated by relatively low levels of Ach staining (Figure 6A). At day 15 post-injection, increased levels of innervation are observed, as indicated by increased levels of Ach staining (Figure 6B). At day 30 post-injection, still more Ach staining is observed (Figure 6C), indicative of further increases in innervation. At 6 months following injection, extensive innervation is observed, as indicated by substantial Ach staining throughout the MDC injected area viewed at low magnification (Figure 6D). These results indicate that the pelvic nerve is growing into the MDC injected area of the bladder, and suggest that the MDC can improve the contractility and function of the injected tissue.

### Contractility physiology studies

To determine whether injected MDC improved the function of the treated bladder tissues, several contractility studies were completed (see above). Table 3 presents the data showing the contractile parameters of bladder muscle following cryoinjury with or without MDC injections.

**Table 3**

| **Group No.** | | **Contraction amplitude (mN/mg)** | | **Velocity(contraction)(mN/s)** | | **No. of specimens** |
|---|---|---|---|---|---|---|
| | | **20Hz/10shocks** | **20Hz/80shock** | **20Hz/10shocks** | **20Hz/80shocks** | |
| **1** | Sham | 0.375±0.24 | 0.697±0.46 | 18.08±8.15 | 15.56±8.39 | 6 |
| | MDC | 0.368±0.26 | 0.812±0.31 | 16.23±10.3 | 16.38±7.54 | 6 |
| **2** | Sham | 0.427±0.17 | 0.966±0.31 | 22.96±8.93 | 24.56±5.03 | 8 |
| | MDC | 0.539±0.24 | 1.161±0.55 | 27.86±14.08 | 30.59±13.05 | 8 |
| **3** | Sham | 0.389±0.14* | 0.708±026** | 25.70±5.87 | 24.24±6.38 | 8 |
| | MDC | 0.564±0.16* | 1.139±0.29** | 30.59±17.8 | 29.31±15.3. | 8 |
| **4** | Normal | 0.927±0.23 | 1.748±0.52 | 34.23±8.82 | 29.05±7.08 | 6 |

| | | | | | | |
|---|---|---|---|---|---|---|
| • p<0.05,** p>0.01 Table 3: Contractile parameters of bladder muscle following cryoinjury. Values are means ± standard deviations. For statistical analysis, Student's test was performed for control and MDC injection groups. Group No. 1: severe damage group with Immediate MDC injections following cryoinjury. Group No. 2: mild damage group with MDC injections one week following cryodamage. Group No. 3: severe damage group with MDC injections one week following cryodamage. Group No. 4: normal bladder tissue. | | | | | | |

The severe damage group injected with MDC Immediately following cryoinjury (Group 1) showed similar contractility as the control (sham) group (Compare contractility levels shown In sham and MDC rows In Group 1, Table 3). However, the severe damage group Injected with MDC one week following the cryodamage (Group 3) showed increased contraction amplitude (145% and 161% of the control bladder at 20Hz/10 shocks and 20Hz/80 shocks, respectively) compared with the control group (compare contractile amplitude levels shown in sham and MDC rows indicated with asterisks in Table 3). Similarly, the severe damage group injected with MDC one week following the cryodamage (Group 3) showed Increased contraction velocity (119% and 121 % of that of the control strip at 20Hz/10 shocks and 20Hz/80 shocks, respectively) compared with the control group (compare contractile velocity values in sham and MDC rows in Group 3, Table 3). The mild damage group injected with MDC one week following the cryodamage (Group 2) also showed increased contraction amplitude and velocity compared to the control group (compare contractility levels shown in sham and MDC rows in Group 2, Table 3). The results of these studies show that MDC injections can restore contractility to cryodamaged bladder muscle tissue, and indicate that MDC-based compositions can be utilized for the treatment of urinary incontinence.

### EXAMPLE 7: Soft tissue augmentation of the myocardium

SD rats were prepared for surgery as described above. A thoracic incision was made to expose the heart. The ventricular wall was injected with 10 µl of MDC suspension in HBSS (1-1.5 x 10⁶ cells) using a Hamilton microsyringe. At day 3, the area surrounding each injection site was excised, prepared for histochemical analysis, stained for β-galactosidase to determine the location and viability of the cells carrying the LacZ marker, examined microscopically, and photographed. The results of these experiments demonstrate that MDC compositions can be used as myocardial soft tissue augmentation materials (Figures 7A and 7B) for the treatment of injury or weakness secondary to heart failure or myocardial infarction.

### EXAMPLE 8: MDC injection into liver, spleen, and spinal cord tissues

SD rats were prepared for surgery as described above. A midline abdomen incision was made to expose the liver and spleen. Both sites were injected with 10 µl of MDC suspension in HBSS (1-1.5 x 10⁶ cells) using a Hamilton microsyringe. At the same time, a midline back incision and a partial laminectomy was made to expose the spinal cord. Spinal cord tissues at level T10 were then injected with the MDC suspension in HBSS as done for the liver and spleen tissues. At day 4, the area surrounding each injection site was excised, prepared histochemical analysis, stained for β-galactosidase to determine the location and viability of the cells carrying the LacZ marker, examined microscopically, and photographed. These experiments show that MDC compositions can be used as liver, spleen, and spinal cord soft tissue augmentation materials (Figures 8A-8B, 9A-9B, and 10A-10B) to treat various liver, spleen, and spinal cord injuries, diseases, or dysfunctions.

### EXAMPLE 9: MDC treatment of bone defects

### Isolation of muscle derived cells

MDC were obtained from *mdx* mice as described in Example 1.

### Clonal isolation of PP6 muscle-derived progenitor cells

To isolate clones from the PP6 cell population, PP6 cells were transfected with a plasmid containing the LacZ, mini-dystrophin, and neomycin resistance genes. Briefly, a *Sma*I/*Sal*I fragment containing the neomycin resistance gene from pPGK-NEO was inserted into the *Sma*I/*Sal*I site in pIEPlacZ plasmid containing the LacZ gene, creating the pNEOlacZ plasmid. The *Xho*I/*Sal*I fragment from DysM3 which contains the short version of the dystrophin gene (K. Yuasa et al., 1998, FEBS Lett. 425:329-336; gift from Dr. Takeda, Japan) was inserted into *Sal*I site in the pNEOlacZ to generate a plasmid which contains the mini-dystrophin, LacZ, and neomydn resistance genes. The plasmid was linearized by *Sal*I digestion prior to transfection.

PP6 cells were transfected with 10 µg of the linear plasmid containing mini-dystrophin, LacZ, and neomycin resistance gene using the Lipofectamine Reagent (Gibco BRL) according to the manufacturers instructions. At 72 hours after transfection, cells were selected with 3000 µg/ml of G418 (Gibco BRL) for 10 days until discrete colonies appeared. Colonies were then isolated and expanded to obtain a large quantity of the transfected tells, and then tested for expression of LacZ. One of these PP6-derived clones, mc13, was used for further study.

### Immunohistochemistry

PP6, mc13, and mouse fibroblast cells were plated in a 6-well culture dish and fixed with cold methanol for 1 minute. Cells were then washed with phosphate buffered saline (PBS), and blocked with 5% horse serum at room temperature for 1 hour. The primary antibodies were diluted in PBS as follows: anti-desmin (1:100, Sigma), biotinylated anti-mouse CD34 (1:200, Pharmingen), rabbit anti-mouse Bcl-2 (1:500, Pharmingen), rabbit anti-mouse M-cadherin (1:50, gift from Dr. A. Wemig), mouse anti-mouse MyoD (1:100, Pharmingen), mouse anti-rat myogenin (1:100, Pharmingen), rabbit anti-mouse Fik-1 (1:50, Research Diagnostics), and biotinylated Sca-1 (1:100, Pharmingen). Cells were incubated with the primary antibodies at room temperature overnight cells were then washed and Incubated with the appropriate biotinylated secondary antibodies for 1 hour at room temperature. Subsequently, the cells were rinsed with PBS then incubated at room temperature with 1/300 streptavidin conjugated with Cy3 fluorochrome for 1 hour. Cells were then analyzed by fluorescence microscopy. For each marker, the percentage of stained cells was calculated for 10 randomly chosen fields of cells.

Cryosections of muscle samples from a four week old normal mouse (C-57 BL/6J, Jackson Laboratories) were fixed with cold acetone for 2 minutes and pre-incubated In 5% horse serum diluted in PBS for 1 hour. For CD34, Bcl-2, and collagen type IV, the following primary antibodies were used: biotin anti-mouse CD34 (1:200 in PBS, Pharmingen), rabbit anti-mouse Bcl-2 (1:1000, Pharmingen), and rabbit anti-mouse collagen type IV. (1:100 In PBS, Chemicon). For dystrophin staining, sheep-anti-human DY10 antibody(1:250 dilution in PBS) was used as the primary antibody, and the signal was amplified using anti-sheep-biotin (1:250 dilution in PBS), and streptavidin-FITC (1:250 dilution in PBS).

### Stimulation with rhBMP-2, osteocalcin staining, and alkaline phosphatase assay

Cells were plated In triplicate at a density of 1-2 x 10⁴ cells per well in 12 well collagen-coated flasks. The cells were stimulated by the addition of 200 ng/ml recombinant human BMP-2 (rhBMP-2) to the growth medium. The growth medium was changed on days 1, 3, and 5 following the initial plating. A control group of cells was grown in parallel without added rhBMP-2. After 6 days with or without rhBMP-2 stimulation, cells were counted using a microcytometer and analyzed for osteocalcin and alkaline phosphatase expression. For osteocalcin staining, cells were incubated with goat anti-mouse osteocalcin antibodies (1:100 in PBS, Chemicon), followed by incubation with anti-goat antibodies conjugated with the Cy3 fluorochrome. To measure alkaline phosphatase activity, cell lysates were prepared and analyzed using a commercially available kit that utilizes color change in the reagent due to the hydrolysis of inorganic phosphate from p-nitrophenyl phosphate (Sigma). The resulting color change was measured on a spectrophotometer, and the data wore expressed as international units ALP activity per liter normalized to 10⁶cells. Statistical significance was analyzed using student's t-test (p< 0.05).

### In vivo differentiation of mc13 cells in myogenic and osteogenic lineages-Myogenic

The mc13 cells (5 x 10⁵ cells) were injected intramuscularly in the hind limb muscle of *mdx* mice. The animals were sacrificed at 15 days post-injection, and the injected muscle tissue was frozen, cryostat sectioned, and assayed for dystrophin (see above) and LacZ expression. To test for LacZ expression, the muscle sections were fixed with 1% glutaraldehyde and then were incubated with X-gal substrate (0.4 mg/ml 5-bromochloro-3 indolyl-β-D-galactoside (Boehringer-Mannheim), 1 mM MgCl₂, 5 mM K₄Fe(CN)₆, and 5 mM K₃Fe(CN)₆ in phosphate buffered saline) for 1-3 hours. Sections were counter-stained with eosin prior to analysis. In parallel experiments, mc13 cells (5 x 10⁵ cells) were injected intravenously in the tall vein of *mdx* mice. The animals were sacrificed at 7 days post-injection and hind limbs were isolated and assayed for the presence of dystrophin and β-galactosidase as described.

### Osteogenic

To construct the adenovirus BMP-2 plasmid (adBMP-2), the rhBMP-2 coding sequence was excised from the BMP-2-125 plasmid (Genetics Institute, Cambridge, MA) and subcloned into a replication defective (E1 and E3 gene deleted) adenoviral vector containing the HuCMV promoter. Briefly, the BMP-2-125 plasmid was digested with Sall, resulting In a 1237 base pair fragment containing the rhBMP-2 cDNA. The rhBMP-2 cDNA was then inserted into the Sall site of the pAd.lox plasmid, which placed the gene under the control of the HuCMV promoter. Recombinant adenovirus was obtained by co-transfection of pAd.lox with psi-5 viral DNA into CRE-8 cells. The resulting adBMP-2 plasmid was stored at -80°C until further use.

Mc13 cells were trypsinized and counted using a microcytometer prior to infection. Cells were washed several times using HBSS (GibcoBRL). Adenovirus particles equivalent to 50 multiplicity of infection units were premixed into HBSS then layered onto the cells. Cells were incubation at 37°C for 4 hours, and then incubated with an equal volume of growth medium, injections of 0.5-1.0x10⁶ cells were performed using a 30-gauge needle on a gas-tight syringe into exposed triceps surae of SCID mice (Jackson Laboratories). At 14-15 days, the animals were anesthetized with methoxyflurane and sacrificed by cervical dislocation. The hind limbs were analyzed by radiography. Subsequently, the triceps surae were isolated and flash frozen In 2-methylbutane buffered In phosphate buffered saline, and pre-cooled in liquid nitrogen. The frozen samples were cut into 5-10 µm sections using a cryostat (Microm, HM 505 E, Fisher scientific) and stored at - 20°C for further analysis.

### RT-PCR analysis

Total RNA was isolated using TRIzol reagent (Life Technologies). Reverse transcription was carried out using SuperScript^{™} Preamplification System for First Strand cDNA Synthesis (Life Technologies) according to the instructions of the manufacturer. Briefly, 100 ng random hexamers were annealed to 1 µg total RNA at 70°C for 10 minutes, and then chilled on ice. Reverse transcription was carried out with 2 µl 10 X PCR buffer, 2 µl 25 mM MgCl₂, 1 µl 10 mM dNTP mix, 2 µl 0.1 M DTT, and 200 U superscript II reverse transcriptase. The reaction mixture was incubated for 50 minutes at 42°C.
polymerase chain reaction (PCR) amplification of the targets was performed in 50 µl reaction mixture containing 2 µl of reverse transcriptase reaction product, 100 µl (5 U) Taq DNA polymerase (Life Technologies), and 1.5 mM MgCl₂. The CD34 PCR primers were designed using Oligo software and had the following sequences: CD34 UP: taa ctt gac ttc tgc tac ca (SEQ ID NO:1); and CD34 DOWN: gtg gtc tta ctg ctg tcc tg (SEQ ID NO:2). The other primers were designed according to previous studies (J. Rohwedel et al., 1995, Exp. Cell Res. 220:92-100; D.D. Cornelison et al., 1997, Dev. Biol. 191:270-283), and had the following sequences: C-MET UP: gaa tgt cgt oct aca cgg cc (SEQ ID NO:3); C-MET DOWN: cac tac aca gtc agg aca ctg c (SEQ ID NO:4); MNF UP: tac ttc ate aaa gtc cct cgg tc (SEQ ID NO:5); MNF DOWN: gta etc tgg aac aga ggc taa ctt (SEQ ID NO:6); BCL-2 UP: agc cct gtg cca cca tgt gtc (SEQ ID NO:7); BCL-2 DOWN: ggc agg ttt gtc gac ctc act (SEQ ID NO:8); MYOGENIN UP: caa cca gga gga gcg cga tct ccg (SEQ ID NO:9); MYOGENIN DOWN: agg cgc tgt ggg agt tgc att cac t (SEQ ID NO:10); MYOD UP: gct ctg atg gca tga tgg att aca gcg (SEQ ID NO:11); and MYOD DOWN: atg ctg gac agg cag tcg agg-c (SEQ ID NO:12).

The following PCR parameters were used: 1) 94°C for 45 seconds; 2) 50°C for 60 seconds (CD34) or 60°C for 60 seconds (for myogenin and c-met); and 3) 72°C for 90 seconds for 40 cycles. PCR products were checked by agarose-TBE-ethldium bromide gels. The sizes of the expected PCR products are: 147 bp for CD34; 86 bp for myogenin; and 370 bp for c-met. To exclude the possibility of genomic DNA contamination, two control reactions were completed: 1) parallel reverse transcription In the absence of reverse transcriptase, and 2) amplification of β-actin using an intron-spanning primer set (Clonetech).

### Skull defect assay

Three 6-8 week old female SCID mice (Jackson Laboratories) were used In control and experimental groups. The animals were anesthetized with methoxyflurane and placed prone on the operating table. Using a number 10 blade, the scalp was dissected to expose the skull, and the periosteum was stripped. An approximately 5 mm full-thickness circular skull defect was created using a dental burr, with minimal penetration of the dura. A collagen sponge matrix (Helistat^{™}, Colla-Tec, Inc.) was seeded with 0.5-1.0 x 10⁸ MDC either with or without adBMP-2 transduction, and placed into the skull defect. The scalp was dosed using a 4-0 nylon suture, and the animals were allowed food and activity. After 14 days, the animals were sacrificed, and the skull specimens were observed and then analyzed microscopically. For von Kossa staining, skull specimens were fixed in 4% formaldehyde and then soaked In 0.1 M AgNo₃ solution for 15 minutes. The specimens were exposed to light for at least 15 minutes, washed with PBS, and then stained with hematoxylin and eosin for viewing.

### Fluorescence in situ hybridization using Y-probes

The cryosections were fixed for 10 minutes In 3:1 methanol/glacial acetic acid (v:v) and air dried. The sections were then denatured In 70% formamide In 2 X SSC (0.3 M NaCl, 0.03 M NaCitrate) pH 7.0 at 70°C for 2 minutes. Subsequently, the slides were dehydrated with a series of ethanol washes (70%, 80%, and 95%) for 2 minutes at each concentration. The Y-chromosome specific probe (Y. Fan et al., 1996, Muscle Nerve 19:853-860) was biotinylated using a BioNick kit (Gibco BRL) according to the manufacturers instructions. The biotinylated probe was then purified using a G-50 Quick Spin Column (Boehringer-Mannheim), and the purified probe was lyophilized along with 5 ng/ml of sonicated herring sperm DNA. Prior to hybridization, the probe was re-suspended in a solution containing 50% formamide, 1 X SSC, and 10% dextran sulfate. After denaturation at 75°C for 10 minutes, the probe was placed on the denatured sections and allowed to hybridize overnight at 37°C. After hybridization, the sections were rinsed with 2 X SSC solution pH 7.0 at 72°C for 5 minutes. The sections were then rinsed In BMS solution (0.1 M NaHCO₃, 0.5 M NaCl, 0.5% NP-40, pH 8.0). The hybridized probe was detected with fluorescein labeled avidin (ONCOR, Inc). The nuclei were counter-stained with 10 ng/ml ethidium bromide in Vectashield mounting medium (Vector, Inc).

### Marker analysis of mc13 cells

The biochemical markers expressed by mc13, PP6, and fibroblast cells were analyzed using RT-PCR and immunohistochemistry. Table 4 (below) shows that mc13 cells expressed Flk-1, a mouse homologue of the human KDR gene, which was recently identified as a marker of hematopoietic cells with stem cell-like characteristic (B.L. Ziegler et al., *supra*), but did not express CD34 or CD45. However, other clonal isolates derived from the PP6 MDC of the present invention expressed CD34, as well as other PP6 cell markers. It will be appreciated by those skilled In the art that the procedures described herein can be used to clone out the PP6 muscle-derived progenitor cell population, and obtain clonal isolates that express cell markers characteristic of the muscle-derived progenitor cells. Such clonal isolates can be used in accordance with the methods of the invention. For example, the clonal isolates express progenitor cell-markers, including desmin, CD34, and Bcl-2. Preferably, the clonal isolates also express the Sca-1 and Flk-1 cell markets, but do not express the CD45 or c-Kit cell markers.

**Table 4**

| | **PP6 cells** | | **MC13 cells** | | **Fibroblasts** | |
|---|---|---|---|---|---|---|
| | lmm | RT-PCR | lmm | RT-PCR | lmm | RT-PCR |
| desmin | + | na | + | na | - | na |
| CD34 | + | + | - | - | - | - |
| Bcl-2 | + | na | +/ | na | - | na |
| Fik-1 | + | na | + | na | - | na |
| Sca-1 | + | na | + | na | - | na |
| M-cadherin | -/+ | na | + | na | - | na |
| Myogenin | +/- | + | +/- | + | - | - |
| c-met | na | + | na | + | na | - |
| MNF | na | + | na | + | na | - |
| MyoD | -/+ | + | na | + | na | - |
| c-Kit | - | na | - | na | na | na |
| CD45 | - | na | - | na | na | na |

| | | | | | | |
|---|---|---|---|---|---|---|
| Table 4: Cell markers expressed by *mdx* PP6, *mdx* mc13, and fibroblast cells. Cells were isolated as described above and examined by immunohistochemical analysis. "-" Indicates that 0% of the cells showed expression; "+" indicates that >98% of the cells showed expression; "+/-" indicates that 40-80% of the cells showed expression; "-/+" indicates that 5-30% of the cells showed expression; "na" indicates that the data is not available. | | | | | | |

### In vivo localization of CD34⁺ and Bcl-2⁺ cells

To identify the location of CD34+ and Bcl-2⁺ cells *in vivo,* muscle tissue sections from the triceps surae of normal mice were stained using anti-CD34 and anti-Bcl-2 antibodies. The CD34 positive cells constituted a small population of muscle derived cells (Figure 12A) that were also positive for desmin (Figure 12B). Co-staining the CD34+, desmin+ cells with anti-collagen type IV antibody localized them within the basal lamina (Figures 12B and 12D). As indicated by the arrowheads in Figures 12A-D, small blood vessels were also positive for CD34 and collagen type IV, but did not co-locelize with the nuclear staining. The expression of CD34 by vascular endothelial cells has been shown in previous studies (L. Fina et al., *supra).* The Bcl-2+, desmin+ cells were similarly identified (Figures 12E-12H) and localized within the basal lamina (Figures 12F and 12H). The sections were also stained for M-cadherin to identify the location of satellite cells (Figure 12I). The satellite cells were identified at similar locations as CD34+, desmin+, or Bcl-2+, desmin+ cells (arrow, Figure 12I). However, multiple attempts to co-localize CD34 or Bcl-2 with M-cadherin were unsuccessful, suggesting that M-cadherin expressing cells do not co-express either Bcl-2 or CD34. This is consistent with PP6 cells expressing high levels of CD34 and Bcl-2, but expressing minimal levels of M-cadherin, as disclosed herein.

### In vitro differentiation of clonal muscle progenitor cells into osteogenic lineage

Mc13 cells were assessed for osteogenic differentiation potential by stimulation with rhBMP-2. Cells were plated on 6-Well culture dishes and grown to confluency in the presence or absence of 200 ng/ml rhBMP-2. Within 3-4 days, mc13 cells exposed to rhBMP-2 showed dramatic morphogenic changes compared to cells without rhBMP-2. In the absence of rhBMP-2, mc13 cells began to fuse into multinucleated myotubes (Figure 13A). When exposed to 200 ng/ml rhBMP-2, however, cells remained mononucleated and did not fuse (Figure 13B). When cell density reached > 90% confluency, the untreated culture fused to form multiple myotubes (Figure 13C), while the treated cells became circular and hypertrophic (Figure 13D). Using immunohistochemistry, these hypertrophic cells were analyzed for the expression of osteocalcin. Osteocalcin is a matrix protein that is deposited on bone, specifically expressed by osteoblasts. In contrast to the untreated group, the rhBMP-2 treated hypertrophic cells showed significant expression of osteocalcin (Figure 13E), thus.suggesting that mc13 cells are capable of differentiating into osteoblasts upon exposure to rhBMP-2.

Mc13 Cells were then analyzed for expression of desmin following rhBMP-2 stimulation. Newly isolated mc13 cells showed uniform desmin staining (Figures 14A and 14B). Within 6 days of exposure to rhBMP-2, only 30-40% of mc13 cells showed desmin staining. In the absence of rhBMP-2 stimulation, approximately 90-100% of mc13 cells showed desmin staining (Figure 14C). This result suggests that stimulation of mc13 cells with rhBMP-2 results in the loss of myogenic potential for these cells.

In addition, mc13 cells were analyzed for the expression of alkaline phosphatase following rhBMP-2 stimulation. Alkaline phosphatase has been used as a biochemical marker for osteoblastic differentiation (T. Katagiri et al., 1994, J. Cell Biol., 127:1755-1766). As shown in Figure 14D, alkaline phosphatase expression of mc13 cells was increased more than 600 fold in response to rhBMP-2. PP1-4 cells, used as a control, did not show increased alkaline phosphatase activity in response to rhBMP-2 (Figure 14D). Taken together, these data demonstrate that cells of a PP6 clonal isolate, e.g., mc13 cells, can lose their myogenic markers and differentiate through the osteogenic lineage in response to rhBMP-2 exposure *in vitro.*

### In vivo differentiation of mc13 cells into myogenic and osteogenic lineages

To determine whether mc13 cells were capable of differentiating through the myogenic lineage *in vivo*, the cells were injected into the hind limb muscle tissue of *mdx* mice. The animals were sacrificed 15 days following injection, and their hind limbs were harvested for histological and immunohistochemical analysis. Several myofibers showed LacZ and dystrophin staining in the region surrounding the injection site (Figures 15A and 15B), indicating that mc13 cells can differentiate through the myogenic lineage *in vivo* and enhance muscle regeneration and restore dystrophin in the dystrophic muscle.

In a parallel experiment, mc13 cells were injected intravenously into the tall vein of *mdx* mice. The animals were sacrificed at 7 days post-injection, and the hind limb muscles were harvested for histological and immunohistochemical analysis. Several hind limb muscle cells showed LacZ and dystrophin staining (Figures 15C-15D; see also"*"), suggesting that mc13 cells can be delivered systemically to the target tissue for rescue of dystrophin expression.

To test the pluripotent characteristics of mc13 cells *in vivo,* the cells were transduced with an adenoviral vector encoding rhBMP-2 (adBMP-2). The mc13 cells with adBMP-2 were then injected into hind limbs of SCID mice. The animals were sacrificed at 14 days post-injection, and the hind limbs were removed for histochemical and immunochemical analysis. Enzyme-linked immunosorbent assay (ELISA) analysis of mc13 cells transduced with adBMP-2. showed that infected cells were capable of producing rhBMP-2. Radiographic analysis of hind limbs of injected SCH3 mice revealed robust ectopic bone formation within 14 days of injection (Figure 15E). Histological analysis using LacZ staining of the ectopic bone shows that LacZ positive mc13 cells were uniformly located within the mineralized matrix or lacunae, a typical location where osteoblasts and osteocytes are found (Figure 15F).

To further confirm the role of mc13 in formation of the ectopic bone, the muscle sections were also stained for presence of dystrophin. As shown in Figure 15G, the ectopic bone contained cells highly positive for dystrophin, suggesting that mc13 cells are intimately participating in bone formation. As a control, similar experiments were carried out with fibroblasts. Fibroblasts were found to support robust ectopic bone formation, but the injected cells were uniformly found outside of the bone, and none could be located within the mineralized matrix. This suggests that the fibroblasts are capable of delivering rhBMP-2 to form ectopic bone, but are unable to differentiate into osteoblasts. In this case, the cells participating in mineralization of the ectopic bone are most likely derived from the host tissue. Thus, these results demonstrate that mc13 cells can differentiate Into osteoblasts, both *in vivo* and *in vitro,*

### Enhancement of bone healing by genetically engineered muscle-derive cells

Skull defects (approximately 5 mm) were created in skeletally mature (6-8 Weeks old) female SCID mice using a dental burr as described above. Previous experiments have demonstrated that 5 mm skull defects are "non-healing" (P.H. Krebsbach et al., 1998, Transplantation 66:1272-1278). The skull defect was filled with a collagen sponge matrix seeded with mc13 cells transduced or not transduced with adBMP-2. These mice were sacrificed at 14 days, and the healing of the skull defect was analyzed. As shown In Figure 16A, the control group treated with mc13 cells without rhBMP-2 showed no evidence of healing of the defect. In contrast, the experimental group treated with mc13 cells transduced to express rhBMP-2 showed almost a full closure of the skull defect at 2 weeks (Figure 168). The von Kossa staining, which highlights mineralized bone, showed robust bone formation in the group treated with mc13 cells transduced to express rhBMP-2 (Figure 16D), but minimal bone formation was observed In the control group (Figure 16C).

The area of new bone In the experimental group was analyzed by fluorescence *in situ* hybridization (FISH) with a Y-chromosome specific probe to identify transplanted cells. As shown in Figure 16E, Y-chromosome positive cells were identified within the newly formed bone, indicating active participation of transplanted cells in bone formation under the influence of rhBMP-2. The Y-chromosome negative cells were also identified within the newly formed skull, thus indicating active participation of host-derived cells as well. These results demonstrate that mc13 cells can mediate healing of a "non-healing" bone defect upon stimulation with rhBMP-2, and indicate that the MDC of the present invention can be used in the treatment of bone defects, injuries, or traumas.

### EXAMPLE 10: Non-autologous (Allogeneic) MDSC treatment of bone defects

### Methods

Normal mice (C57 BL/6J), or mdx mice (C57BL/10ScSn mdx/mdx mice) used in the experiments described in this Example were purchased from Jackson Laboratories (Bar Harbor, ME) and used as donors and hosts, respectively.

For preparation of muscle-derived stem cell (MDSC) and satellite cell (scs) cultures, the hindlimb muscles were removed from newborn (3-5 days old) normal mice and the muscle cells were enzymatically dissociated by the addition of 0.2% collagenase-type XI for 1 hour at 37°C. 2.4 units/ml of dispase was added for 45 minutes, and 0.1 % trypsin for 30 minutes. The muscle cell extract was then pre-plated on collagen-coated flasks in proliferation medium (DMEM contained 10% horse serum, 10% fetal bovine serum, 0.5% chick embryo extract, and 1 % penicillin/streptomycin). The scs of early preplates attached to the flask substrate In 1-4 days, while the mdsc took 5-7 days (e.g.,PPS-6), as described.

0.35-0.74 x 10⁶ cells from both the late preplate (LP), (PP5 or PP6) and the early preplate (EP), (PP1-2) were injected into each hindlimb muscle of mdx mice by single point injection. Injected muscles were excised by 10 and 30 days post-transplantation, respectively, and frozen in liquid nitrogen. Cryo-sections were prepared from the injected muscle for immunohistochemical analysis. For immunohistochemical analysis on muscle cross-sections, dystrophin staining was performed. Staining was observed using fluorescence microscopy (Nikon Optiphot), and the number of dystrophin-positive (dystrophin+) cells were counted.

A large engraftment with huge number of dystrophin+ myofibers was observed in the MDSC [LP]-injected muscle by ten days post-injection (Figs. 17C and 17D). Compared with MDSC [EP]-injected muscle (Figs.17A and 17B), the MDSC [LP]-engraftment contained many more small myofibers, thus suggesting that the injected MDSC [LP] cells possess a high proliferative ability *in vivo.* In both muscles, the same number of muscle cells were injected. The number of dystrophin+ myofibers in the muscle injected with MDSC [LP] was about 5 times greater than that in the muscle injected with MDSC [EP] (scs), (2.798 +/- 1.114, n=4 in mdsc vs. 430 +/- 148, n=6 in EP; Mean +/- SD).

In addition, a higher number of dystroiphin+ myofibers were present in the engraftment at 30 days post-injection of MDSC [LP] (Figs. 17E and 17F). As described above, most of the dystrophin+ myofibers that had formed by 10 days post injection survived 20 days later at day 30. It was found that the late plate MDSC (e.g., PP5-6) gave rise to more satellite cells in host muscle than did the early plate cells (e.g., PP1-2), which could contribute to the high number of dystrophin+ myofibers in the late plate-injected muscle. Muscle derived stem cells significantly improved the efficiency of cell transplantation into dystrophic muscle. In addition to the high self renewal ability confirmed in late-plate cells, the MDSC cells bypass immune-rejection, which is likely to be a factor responsible for the high survival rate of late-plate cells in non-autologous host muscle.

### EXAMPLE 11: Augmentation of the Vertebral Disc

Female rabbits were prepared for surgery in the lateral position under halothan anesthesia. A perispinal incision was made in the region of T4-L2 spinal level to expose the discs. The discs were injected with 10 µl of a suspension of muscle-derived progenitor cells, carrying the LacZ marker, In HBSS (1-1.5 x 10⁶ cells) using a Hamilton microsyringe. At day 10 post-injection, the disc was excised, prepared for histochemical analysis, stained for β-galactosidase to determine the location and viability of the cells carrying the LacZ marker, examined microscopically, and photographed. Results of these experiments demonstrate that MDC compositions can be used as disc augmentation materials (Figs. 20A and 20B) for the treatment of congenital, degenerative or traumatic disc symptoms, injury, or conditions including back pain and deficiency of the disc and spinal column.

## Claims

1. Use of a composition comprising (i) isolated, desmin-expressing, muscle-derived progenitor cells (MDC) having long-term survivability *in situ,* or a clonal population thereof, and (ii) a physiologically-acceptable carrier, excipient, or diluent, for the manufacture of a medicament for use in augmenting or bulking esophageal muscle tissue or gastroesophageal muscle tissue in a mammal, wherein the composition is present in an amount sufficient to augment or bulk the esophageal or gastroesophageal muscle tissue.

2. Use of a composition comprising (i) isolated, desmin-expressing, muscle-derived progenitor cells (MDC) having long-term survivability *in situ,* or a clonal population thereof, and (ii) a physiologically-acceptable carrier, excipient, or diluent, for the manufacture of a medicament for use in augmenting or bulking sphincter muscle tissue in a mammal, wherein the composition is present in an amount sufficient to augment or bulk the sphincter muscle tissue.

3. Use of a composition comprising (i) isolated, desmin-expressing, muscle-derived progenitor cells (MDC) having long-term survivability *in situ,* or a clonal population thereof, and (ii) a physiologically-acceptable carrier, excipient, or diluent, for the manufacture of a medicament for use in augmenting or bulking tissue selected from bladder, ureteral, or urethral muscle tissue in a mammal, wherein the composition is present in an amount sufficient to augment or bulk the bladder, ureteral, or urethral tissue.

4. Use of a composition comprising (i) isolated, desmin-expressing, muscle-derived progenitor cells (MDC) having long-term survivability *in situ,* or a clonal population thereof, and (ii) a physiologically-acceptable carrier, excipient, or diluent, for the manufacture of a medicament for use in augmenting or bulking a soft tissue cosmetic or aesthetic defect in a mammal, wherein the composition is present in an amount sufficient to augment or bulk the cosmetic or aesthetic defect.

5. The use according to claim 4, wherein the cosmetic or aesthetic defect is selected from the group consisting of wrinkles, cutaneous depressions of non-traumatic origin, rhytids, stretch marks, depressed scars, scarring from acne vulgaris, hypoplasia of the lip, facial depressions, dermatological lesions and facial depressions around the eyes.

6. Use of a composition comprising (i) isolated, desmin-expressing, muscle-derived progenitor cells (MDC) having long-term survivability *in situ,* or a clonal population thereof, and (ii) a physiologically-acceptable carrier, excipient, or diluent, for the manufacture of a medicament for use in augmenting or bulking tissue comprising one or more of a cutaneous depression, wound, fissure, or opening in a mammal, wherein the composition is present in an amount sufficient to augment or bulk the cutaneous depression, wound, fissure, or opening.

7. The use according to claim 6, wherein the cutaneous depression, wound, fissure, or opening is selected from lesions, diverticulae, cysts, fistulae, aneurysms, and wounds secondary to injury, trauma, surgery, or disease.

8. Use of a composition comprising (i) isolated, desmin-expressing, muscle-derived progenitor cells (MDC) having long-term survivability *in situ,* or a clonal population thereof, and (ii) a physiologically-acceptable carrier, excipient, or diluent, for the manufacture of a medicament for use in augmenting or bulking muscle tissue in a mammal, wherein the composition is present in an amount sufficient to augment or bulk the muscle tissue; said muscle tissue being selected from (a) digestive tissue selected from the group consisting of stomach, intestinal, tongue, esophageal, pyloric and anal tissue; (b) reproductive tissue selected from the group consisting of uterine, vaginal; clitoral, fallopian tube,-cervical, penile and vas deferens tissue; (c) urological tissue selected from the group consisting of kidney, bladder, urethral, ureteral and sphincter tissue; or (d) respiratory tissue selected from the group consisting of tracheal and lung tissue.

9. The use according to claim 8, wherein the augmenting or bulking treats a weakness or dysfunction that is (a) secondary to a sports-related injury; or (b) selected from the group consisting of vesico-ureteral reflux, urinary incontinence, fecal incontinence and gastroesophageal reflux.

10. Use of a composition comprising (i) isolated, desmin-expressing muscle-derived progenitor cells (MDC) having long-term survivability *in situ,* or a clonal population thereof, and (ii) a physiologically-acceptable carrier, excipient, or diluent, for the manufacture of a medicament for use in augmenting or bulking (a) non-muscle soft tissue, or (b) non-muscle, non-bone soft tissue in a mammal, wherein the composition is present in an amount sufficient to augment or bulk the (a) non-muscle soft tissue, or (b) the non-muscle, non-bone soft tissue.

11. The use according to claim 10, wherein the augmentation or bulking results in repair of a defect selected from the group consisting of lesions, fissures, diverticulae, cysts, fistulae, aneurysms, wounds secondary to injury, trauma, surgery and disease in the non-muscle soft tissue, or the non-muscle, non-bone soft tissue.

12. The use according to claim 10, wherein the non-muscle, non-bone soft tissue is a) digestive tissue selected from the group consisting of oral, stomach, intestinal, esophageal, pyloric, pancreatic, liver, gall bladder and anal tissue; (b) reproductive tissue selected from the group consisting of uterine, vaginal, clitoral, fallopian tube, vulval, cervical, penile, scrotum, testes and vas deferens tissue; (c) urological tissue selected from the group consisting of kidney, bladder, urethral, ureteral and sphincter tissue; (d) respiratory tissue selected from the group consisting of tracheal and lung tissue; (e) neural tissue selected from the group consisting of nerve, spinal cord, vertebral disc and brain tissue; and (f) epithelial tissue selected from the group consisting of skin and lumenal tissue.

13. Use of a composition comprising (i) isolated, desmin-expressing muscle-derived progenitor cells (MDC) having long-term survivability *in situ,* or a clonal population thereof, and (ii) a physiologically-acceptable carrier, excipient, or diluent, for the manufacture of a medicament for use in restoring or improving contractility of smooth muscle, wherein the smooth muscle is gastrointestinal smooth muscle selected from the group consisting of eosphagus smooth muscle, stomach smooth muscle, pyloric smooth muscle, and intestine smooth muscle; and further wherein the composition is present in an amount sufficient to restore or improve the smooth muscle contractility.

14. The use according to claim 13, wherein the restoring or improving of the gastrointestinal smooth muscle results in improvement or correction of gastroparesis.

15. The use according to any one of claims 1, 2, 3, 4, 6, 8, 10, or 13, wherein the MDC in the composition of the medicament contain heterologous DNA encoding one or more active biomolecules, and wherein the biomolecules are expressed by the cells, thereby assisting in treatment of the tissue, wherein the active biomolecule is selected from the group consisting of one or more of cell growth factors, cell differentiation factors, cell signaling factors and programmed cell death factors.

16. The use according to any one of claims 1, 2, 3, 4, 6, 8, 10, or 13, wherein the carrier comprises an absorbent or adherent material.

17. The use according to claim 16, wherein the carrier is a collagen sponge material.

18. The use according to any one of claims 1 to 17, wherein the composition further ameliorates or treats weakness or dysfunction in the tissue.

19. The use according to any one of claims 1, 2, 3, 4, 6, 8, 10, or 13, wherein the medicament is capable of being injected into the tissue or administered endoscopically.

20. The use according to any one of claims 1,2,3,4,6, 8,10, or 13, wherein the composition comprises MDC which are autologous to the tissue.

21. The use according to any one of claims 1, 2, 3, 4, 6, 8,10, or 13, wherein the composition comprises MDC which are allogeneic to the tissue.

22. The use according to claim 20 or claim 21, wherein the composition comprises MDC obtained from a human source.

## Patentansprüche

1. Verwendung einer Zusammensetzung umfassend (i) isolierte, Desmin exprimierende, muskelderivierte Vorläuferzellen (MDC), die eine Langzeitüberlebensfähigkeit in situ aufweisen, oder eine klonale Population derselben und (ii) einen physiologisch akzeptablen Träger, ein physiologisch akzeptables Vehikel oder Verdünnungsmittel für die Herstellung eines Medikaments zur Verwendung zum Verstärken oder Verdicken des ösophagealen Muskelgewebes oder gastroösophagealen Muskelgewebes bei einem Säuger, wobei die Zusammensetzung in einer Menge vorliegt, die ausreicht, um das ösophageale oder gastroösophageale Muskelgewebe zu verstärken oder zu verdicken.

2. Verwendung einer Zusammensetzung umfassend (i) isolierte, Desmin exprimierende, muskelderivierte Vorläuferzellen (MDC), die eine Langzeitüberlebensfähigkeit in situ aufweisen, oder eine klonale Population derselben und (ii) einen physiologisch akzeptablen Träger, ein physiologisch akzeptables Vehikel oder Verdünnungsmittel für die Herstellung eines Medikaments zur Verwendung zum Verstärken oder Verdicken des Sphinktermuskelgewebes bei einem Säuger, wobei die Zusammensetzung in einer Menge vorliegt, die ausreicht, um das Sphinktermuskelgewebe zu verstärken oder zu verdicken.

3. Verwendung einer Zusammensetzung umfassend (i) isolierte, Desmin exprimierende, muskelderivierte Vorläuferzellen (MDC), die eine Langzeitüberlebensfähigkeit in situ aufweisen, oder eine klonale Population derselben und (ii) einen physiologisch akzeptablen Träger, ein physiologisch akzeptables Vehikel oder Verdünnungsmittel für die Herstellung eines Medikaments zur Verwendung zum Verstärken oder Verdicken von Gewebe ausgewählt unter Blase, Harnröhre oder Harnröhrenmuskelgewebe bei einem Säuger, wobei die Zusammensetzung in einer Menge vorliegt, die ausreicht, um das Blasen-, Harnröhren- oder Harnröhrenmuskelgewebe zu verstärken oder zu verdicken.

4. Verwendung einer Zusammensetzung umfassend (i) isolierte, Desmin exprimierende, muskelderivierte Vorläuferzellen (MDC), die eine Langzeitüberlebensfähigkeit in situ aufweisen, oder eine klonale Population derselben und (ii) einen physiologisch akzeptablen Träger, ein physiologisch akzeptables Vehikel oder Verdünnungsmittel für die Herstellung eines Medikaments zur Verwendung zum Verstärken oder Verdicken einer kosmetischen oder ästhetischen Weichteildefektstelle bei einem Säuger, wobei die Zusammensetzung in einer Menge vorliegt, die ausreicht, um die kosmetische oder ästhetische Defektstelle zu verstärken oder zu verdicken.

5. Verwendung nach Anspruch 4, wobei die kosmetische oder ästhetische Defektstelle aus der Gruppe ausgewählt ist bestehend aus Runzeln, Hautvertiefungen nichttraumatischen Ursprungs, Rhytiden, Schwangerschaftsstreifen, eingedrückten Narben, durch Akne vulgaris verursachten Narben, Unterentwicklung der Lippe, Gesichtsvertiefungen, dermatologischen Läsionen und Gesichtsvertiefungen um die Augen.

6. Verwendung einer Zusammensetzung umfassend (i) isolierte, Desmin exprimierende, muskelderivierte Vorläuferzellen (MDC), die eine Langzeitüberlebensfähigkeit in situ aufweisen, oder eine klonale Population derselben und (ii) einen physiologisch akzeptablen Träger, ein physiologisch akzeptables Vehikel oder Verdünnungsmittel für die Herstellung eines Medikaments zur Verwendung zum Verstärken oder Verdicken von Gewebe umfassend eine oder mehrere Hautvertiefungen, Wunden, Risse oder Öffnungen bei einem Säuger, wobei die Zusammensetzung in einer Menge vorliegt, die ausreicht, um die Hautvertiefung, Wunde, den Riss oder die Öffnung zu verstärken oder zu verdicken.

7. Verwendung nach Anspruch 6, wobei die Hautvertiefung, Wunde, der Riss oder die Öffnung ausgewählt sind unter Läsionen, Divertikeln, Zysten, Fisteln, Aneurismen und Wunden als Folge von Verletzung, Trauma, operativem Eingriff oder Krankheit.

8. Verwendung einer Zusammensetzung umfassend (i) isolierte, Desmin exprimierende, muskelderivierte Vorläuferzellen (MDC), die eine Langzeitüberlebensfähigkeit in situ aufweisen, oder eine klonale Population derselben und (ii) einen physiologisch akzeptablen Träger, ein physiologisch akzeptables Vehikel oder Verdünnungsmittel für die Herstellung eines Medikaments zur Verwendung zum Verstärken oder Verdicken von Muskelgewebe bei einem Säuger, wobei die Zusammensetzung in einer Menge vorliegt, die ausreicht, um das Muskelgewebe zu verstärken oder zu verdicken, wobei das Muskelgewebe ausgewählt ist unter (a) Verdauungsgewebe ausgewählt aus der Gruppe bestehend aus Magen-, Darm-, Zungen-, Speiseröhre-, Magenkranz- und Analgewebe; (b) Geschlechtsorgangewebe ausgewählt aus der Gruppe bestehend aus Gebärmutter-, Vaginal-, Klitoris-, Eileiter-, Gebärmutterhals-, Penis- und Samenleitergewebe; (c) urologischem Gewebe ausgewählt aus der Gruppe bestehend aus Niere, Blase, Harnröhre, ureterischem und Sphinktergewebe; oder (d) Atemwegsgewebe ausgewählt aus der Gruppe bestehend aus Luftröhren- oder Lungengewebe.

9. Verwendung nach Anspruch 8, wobei das Verstärken und Verdicken der Behandlung einer Schwäche oder Funktionsstörung dient, die (a) eine Folge einer sportbedingten Verletzung ist; oder (b) aus der Gruppe ausgewählt ist bestehend aus vesiko-ureterischem Rückfluss, Harninkontinenz, Stuhlinkontinenz und gastroösophagealem Rückfluss.

10. Verwendung einer Zusammensetzung umfassend (i) isolierte, Desmin exprimierende, muskelderivierte Vorläuferzellen (MDC), die eine Langzeitüberlebensfähigkeit in situ aufweisen, oder eine klonale Population derselben und (ii) einen physiologisch akzeptablen Träger, ein physiologisch akzeptables Vehikel oder Verdünnungsmittel für die Herstellung eines Medikaments zur Verwendung zum Verstärken oder Verdicken (a) von Nichtmuskelweichteil oder (b) Nichtmuskel-Nichtknochenweichteil bei einem Säuger, wobei die Zusammensetzung in einer Menge vorliegt, die ausreicht, um (a) das Nichtmuskelgewebe oder (b) das Nichtknochen-Nichtmuskelgewebe zu verstärken oder zu verdicken.

11. Verwendung nach Anspruch 10, wobei das Verstärken oder Verdicken zur Reparatur einer Defektstelle führt ausgewählt aus der Gruppe bestehend aus Läsionen, Rissen, Divertikeln, Zysten, Fisteln, Aneurismen, Wunden als Folge von Verletzung, Trauma, operativem Eingriff und Krankheit im Nichtmuskelweichteil oder dem Nichtmuskel-Nichtknochenweichteil.

12. Verwendung nach Anspruch 10, wobei das Nichtmuskel-Nichtknochenweichteil a) Verdauungsgewebe ist ausgewählt aus der Gruppe bestehend aus Mund-, Darm-, Speiseröhre-, Magenkranz-, Pankreas-, Leber-, Gallenblasen- und Analgewebe; (b) Geschlechtsorgangewebe ausgewählt aus der Gruppe bestehend aus Gebärmutter-, Vaginal-, Klitoris-, Eileiter-, Vulva-, Gebärmutterhals-, Penis-, Hodensack-, Hoden- und Samenleitergewebe; (c) urologischem Gewebe ausgewählt aus der Gruppe bestehend aus Niere, Blase, Harnröhre, ureterischem und Sphinktergewebe; (d) Atemwegsgewebe ausgewählt aus der Gruppe bestehend aus Luftröhren- und Lungengewebe; (e) Nervengewebe ausgewählt aus der Gruppe bestehend aus Nerv, Rückenmark, Wirbelscheibe und Hirngewebe; und (f) Epithelgewebe ausgewählt aus der Gruppe bestehend aus Haut und Lumengewebe.

13. Verwendung einer Zusammensetzung umfassend (i) isolierte, Desmin exprimierende, muskelderivierte Vorläuferzellen (MDC), die eine Langzeitüberlebensfähigkeit in situ aufweisen, oder eine klonale Population derselben und (ii) einen physiologisch akzeptablen Träger, ein physiologisch akzeptables Vehikel oder Verdünnungsmittel für die Herstellung eines Medikaments zur Verwendung zum Wiederherstellen oder Verbessern der Kontraktilität glatter Muskeln, wobei der glatte Muskel ein Magen-Darm-Muskel ist ausgewählt aus der Gruppe bestehend aus einem glatten Speiseröhremuskel, glatten Magenmuskel, glatten Magenkranzmuskel und glatten Darmmuskel; und wobei des Weiterem die Zusammensetzung in einer Menge vorliegt, die ausreicht, um die Kontraktilität des glatten Muskels wiederherzustellen oder zu verbessern.

14. Verwendung nach Anspruch 13, wobei das Wiederherstellen oder Verbessern des Magen-Darm-Muskels zum Bessern oder zur Korrektur von Gastroparese führt.

15. Verwendung nach einem der Ansprüche 1, 2, 3, 4, 6, 8, 10 oder 13, wobei die MDC in der Zusammensetzung des Medikaments heterologe DNA enthalten, die ein oder mehrere aktive Biomoleküle kodiert und wobei die Biomoleküle durch die Zellen exprimiert werden, wodurch die Behandlung des Gewebes unterstützt wird, wobei das aktive Biomolekül aus der Gruppe ausgewählt ist bestehend aus einem oder mehreren Zellwachstumsfaktoren, Zelldifferentierungsfaktoren, Zellsignalisierungsfaktoren und programmierten Zelltotfaktoren.

16. Verwendung nach einem der Ansprüche 1, 2, 3, 4, 6, 8, 10 oder 13, wobei der Träger ein absorptionsfähiges oder haftendes Material umfasst.

17. Verwendung nach Anspruch 16, wobei der Träger Kollagenschwammmaterial ist.

18. Verwendung nach einem der Ansprüche 1 bis 17, wobei die Zusammensetzung des Weiteren eine Schwäche oder Funktionsstörung im Gewebe bessert oder behandelt.

19. Verwendung nach einem der Ansprüche 1, 2, 3, 4, 6, 8, 10 oder 13, wobei das Medikament in der Lage ist, in das Gewebe eingespritzt oder endoskopisch verabreicht zu werden.

20. Verwendung nach einem der Ansprüche 1, 2, 3, 4, 6, 8, 10 oder 13, wobei die Zusammensetzung MDC umfasst, die zum Gewebe autolog sind.

21. Verwendung nach einem der Ansprüche 1, 2, 3, 4, 6, 8, 10 oder 13, wobei die Zusammensetzung MDC umfasst, die zum Gewebe allogen sind.

22. Verwendung nach Anspruch 20 oder Anspruch 21, wobei die Zusammensetzung MDC umfasst, die aus einer menschlichen Quelle erhalten werden.

## Revendications

1. Utilisation d'une composition comprenant (i) des cellules souches dérivées du muscle (MDC) qui expriment la desmine isolées et capables de survivre à long terme in situ ou une population clonale de cellules de ce type et (ii) un véhicule, excipient ou diluant physiologiquement acceptable, dans la fabrication d'un médicament conçu pour produire la croissance ou le comblement d'un tissu musculaire oesophagien ou gastro-oesophagien chez un mammifère et dans lequel la composition est présente en une quantité suffisante pour produire la croissance ou le comblement d'un tissu musculaire oesophagien ou gastro-oesophagien.

2. Utilisation d'une composition comprenant (i) des cellules souches dérivées du muscle (MDC) qui expriment la desmine isolées et capables de survivre à long terme in situ ou une population clonale de cellules de ce type et (ii) un véhicule, excipient ou diluant physiologiquement acceptable, dans la fabrication d'un médicament conçu pour produire la croissance ou le comblement d'un tissu musculaire sphinctérien chez un mammifère et dans lequel la composition est présente en une quantité suffisante pour produire la croissance ou le comblement d'un tissu musculaire sphinctérien.

3. Utilisation d'une composition comprenant (i) des cellules souches dérivées du muscle (MDC) qui expriment la desmine isolées et capables de survivre à long terme in situ ou une population clonale de cellules de ce type et (ii) un véhicule, excipient ou diluant physiologiquement acceptable, dans la fabrication d'un médicament conçu pour produire la croissance ou le comblement d'un tissu musculaire vésical, urétéral ou urétral chez un mammifère et dans lequel la composition est présente en une quantité suffisante pour produire la croissance ou le comblement d'un tissu musculaire vésical, urétéral ou urétral.

4. Utilisation d'une composition comprenant (i) des cellules souches dérivées du muscle (MDC) qui expriment la desmine isolées et capables de survivre à long terme in situ ou une population clonale de cellules de ce type et (ii) un véhicule, excipient ou diluant physiologiquement acceptable, dans la fabrication d'un médicament conçu pour produire la croissance ou le comblement d'un tissu mou siège d'une imperfection cosmétique ou esthétique chez un mammifère et dans lequel la composition est présente en une quantité suffisante pour produire la croissance ou le comblement d'un tissu mou siège d'une imperfection cosmétique ou esthétique.

5. Utilisation selon la revendication 4, dans laquelle l'imperfection cosmétique ou esthétique est sélectionnée dans le groupe constitué par des rides, des dépressions cutanées d'origine non traumatique, des zones de relâchement de la peau, des vergetures, des cicatrices déprimées, des séquelles cicatricielles de l'acné vulgaire, une hypoplasie du muscle de la lèvre, des dépressions faciales, des lésions dermatologiques et des dépressions faciales périoculaires.

6. Utilisation d'une composition comprenant (i) des cellules souches dérivées du muscle (MDC) qui expriment la desmine isolées et capables de survivre à long terme in situ ou une population clonale de cellules de ce type et (ii) un véhicule, excipient ou diluant physiologiquement acceptable, dans la fabrication d'un médicament conçu pour produire la croissance ou le comblement d'un tissu qui comporte une ou plusieurs défectuosités consistant en une dépression cutanée, plaie, fissure ou brèche chez un mammifère et dans lequel la composition est présente en une quantité suffisante pour produire la croissance ou le comblement d'un tissu qui comporte une dépression cutanée, plaie, fissure ou brèche.

7. Utilisation selon la revendication 6, dans laquelle la dépression cutanée, plaie, fissure ou brèche est sélectionnée dans le groupe constitué par des lésions, diverticules, kystes, fistules, anévrismes et plaies secondaires à une blessure, un traumatisme, un acte chirurgical ou une pathologie.

8. Utilisation d'une composition comprenant (i) des cellules souches dérivées du muscle (MDC) qui expriment la desmine isolées et capables de survivre à long terme in situ ou une population clonale de cellules de ce type et (ii) un véhicule, excipient ou diluant physiologiquement acceptable, dans la fabrication d'un médicament conçu pour produire la croissance ou le comblement d'un tissu musculaire chez un mammifère et dans lequel la composition est présente en une quantité suffisante pour produire la croissance ou le comblement d'un tissu musculaire, ledit tissu musculaire correspondant à (a) un tissu digestif sélectionné dans le groupe constitué par un tissu de l'estomac, de l'intestin, de la langue, de l'oesophage, du pylore et de l'anus, (b) un tissu reproducteur sélectionné dans le groupe constitué par un tissu de l'utérus, du vagin, du clitoris, des trompes de Fallope, du col, du pénis et du canal déférent, (c) un tissu urologique sélectionné dans le groupe constitué par un tissu du rein, de la vessie, de l'urètre, de l'uretère et d'un sphincter ou (d) un tissu respiratoire sélectionné dans le groupe constitué par un tissu de la trachée et du poumon.

9. Utilisation selon la revendication 8, dans laquelle la croissance ou le comblement traite une faiblesse ou une dysfonction qui est (a) secondaire à un traumatisme sportif ou (b) sélectionnée dans le groupe constitué par un reflux vésico-urétéral, une incontinence urinaire, une incontinence anale et un reflux gastro-oesophagien.

10. Utilisation d'une composition comprenant (i) des cellules souches dérivées du muscle (MDC) qui expriment la desmine isolées et capables de survivre à long terme in situ ou une population clonale de cellules de ce type et (ii) un véhicule, excipient ou diluant physiologiquement acceptable, dans la fabrication d'un médicament conçu pour produire la croissance ou le comblement (a) d'un tissu mou non musculaire ou (b) d'un tissu mou non musculaire et non osseux chez un mammifère et dans lequel la composition est présente en une quantité suffisante pour produire la croissance ou le comblement (a) d'un tissu mou non musculaire ou (b) d'un tissu mou non musculaire et non osseux.

11. Utilisation selon la revendication 10, dans laquelle la croissance ou le comblement résulte en la réparation d'une défectuosité sélectionnée dans le groupe constitué par des lésions, fissures, diverticules, kystes, fistules, anévrismes et plaies secondaires à une blessure, un traumatisme un acte chirurgical ou une pathologie ayant pour siège un tissu mou non musculaire ou un tissu mou non musculaire et non osseux.

12. Utilisation selon la revendication 10, dans laquelle le tissu mou non musculaire et non osseux correspond à a) un tissu digestif sélectionné dans le groupe constitué par un tissu de la cavité buccale, de l'estomac, de l'intestin, de l'oesophage, du pylore, du pancréas, du foie, de la vésicule biliaire et de l'anus, (b) un tissu reproducteur sélectionné dans le groupe constitué par un tissu de l'utérus, du vagin, du clitoris, des trompes de Fallope, de la vulve, du col, du pénis, du scrotum, des testicules et du canal déférent, (c) un tissu urologique sélectionné dans le groupe constitué par un tissu du rein, de la vessie, de l'urètre, de l'uretère et d'un sphincter, (d) un tissu respiratoire sélectionné dans le groupe constitué par un tissu de la trachée et du poumon, (e) un tissu neural sélectionné dans le groupe constitué par un tissu d'un nerf, de la moelle épinière, d'un disque intervertébral et du cerveau et (f) un tissu épithélial sélectionné dans le groupe constitué par un tissu de la peau et d'une lumière.

13. Utilisation d'une composition comprenant (i) des cellules souches dérivées du muscle (MDC) qui expriment la desmine isolées et capables de survivre à long terme in situ ou une population clonale de cellules de ce type et (ii) un véhicule, excipient ou diluant physiologiquement acceptable, dans la fabrication d'un médicament conçu pour rétablir ou améliorer la contractilité d'un muscle lisse, dans laquelle le muscle lisse est un muscle lisse gastro-intestinal sélectionné dans le groupe constitué par un muscle lisse oesophagien, un muscle lisse gastrique, un muscle lisse pylorique et un muscle lisse intestinal et dans laquelle la composition est présente en une quantité suffisante pour rétablir ou améliorer la contractilité du muscle lisse.

14. Utilisation selon la revendication 13, dans laquelle le rétablissement ou l'amélioration de la contractilité du muscle lisse gastro-intestinal résulte en l'amélioration ou la correction d'une gastroparésie.

15. Utilisation selon l'une quelconque des revendications 1, 2, 3, 4, 6, 8, 10 ou 13, dans laquelle les MDC qui entrent dans la composition du médicament contiennent un ADN hétérologue codant pour une ou plusieurs biomolécules actives qui sont exprimées par les cellules et interviennent ainsi dans le traitement du tissu, lesdites biomolécules actives étant sélectionnées dans le groupe constitué par des facteurs de croissance cellulaire, des facteurs de différenciation cellulaire, des facteurs de signalisation cellulaire et des facteurs d'apoptose.

16. Utilisation selon l'une quelconque des revendications 1, 2, 3, 4, 6, 8,10 ou 13, dans laquelle le véhicule comprend un matériau absorbant ou adhésif.

17. Utilisation selon la revendication 16, dans laquelle le véhicule est un matériau spongieux à base de collagène.

18. Utilisation selon l'une quelconque des revendications 1 à 17, dans laquelle la composition permet également d'améliorer ou de traiter une faiblesse ou une dysfonction dans le tissu.

19. Utilisation selon l'une quelconque des revendications 1, 2, 3, 4, 6, 8, 10 ou 13, dans laquelle le médicament peut être injecté dans le tissu ou administré par voie endoscopique.

20. Utilisation selon l'une quelconque des revendications 1, 2, 3, 4, 6, 8, 10 ou 13, dans laquelle la composition comprend des MDC qui sont autologues par rapport au tissu.

21. Utilisation selon l'une quelconque des revendications 1, 2, 3, 4, 6, 8, 10 ou 13, dans laquelle la composition comprend des MDC qui sont allogéniques par rapport au tissu.

22. Utilisation selon la revendication 20 ou la revendication 21, dans laquelle la composition comprend des MDC d'origine humaine.


### SEQUENCE LISTING

<110> University of Pittsburgh
   200 Gardner Steel Conference Center
   Thackeray & O'Hara Streets
   Pittsburgh, PA 15260
<120> SOFT TISSUE AND BONE AUGMENTATION AND BULKING UTILIZING MUSCLE-DERIVED PROGENITOR CELLS, COMPOSITIONS AND TREATMENTS THEREOF
<130> 27104007PC2
<140> TO BE ASSIGNED
   <141> 2001-04-12
<150> US09/549,937
   <151> 2000-04-14
<160> 12
<170> PatentIn Ver. 2.1
<210> 1
   <211> 20
   <212> DNA
   <213> Artificial Sequence
<220>
   <223> Description of Artificial Sequence: CD34 UP OLIGONUCLEOTIDE SEQUENCE
<400> 1
   taacttgact tctgctacca 20
<210> 2
   <211> 20
   <212> DNA
   <213> Artificial Sequence
<220>
   <223> Description of Artificial Sequence: CD34 DOWN OLIGONUCLEOTIDE SEQUENCE
<400> 2
   gtggtcttac tgctgtcctg 20
<210> 3
   <211> 20
   <212> DNA
   <213> Artificial Sequence
<220>
   <223> Description of Artificial Sequence: C-MET UP OLIGONUCLEOTIDE SEQUENCE
<400> 3
   gaatgtcgtc ctacacggcc 20
<210> 4
   <211> 22
   <212> DNA
   <213> Artificial Sequence
<220>
   <223> Description of Artificial Sequence: C-MET DOWN OLIGONUCLEOTIDE SEQUENCE
<400> 4
   cactacacag tcaggacact gc 22
<210> 5
   <211> 23
   <212> DNA
   <213> Artificial Sequence
<220>
   <223> Description of Artificial Sequence: MNF UP OLIGONUCLEOTIDE SEQUENCE
<400> 5
   tacttcatca aagtccctcg gtc 23
<210> 6
   <211> 24
   <212> DNA
   <213> Artificial Sequence
<220>
   <223> Description of Artificial Sequence: MNF DOWN OLIGONUCLEOTIDE SEQUENCE
<400> 6
   gtactctgga acagaggcta actt 24
<210> 7
   <211> 21
   <212> DNA
   <213> Artificial Sequence
<220>
   <223> Description of Artificial Sequence: BCL-2 UP OLIGONUCLEOTIDE SEQUENCE
<400> 7
   agccctgtgc caccatgtgt c 21
<210> 8
   <211> 21
   <212> DNA
   <213> Artificial Sequence
<220>
   <223> Description of Artificial Sequence: BCL-2 DOWN OLIGONUCLEOTIDE SEQUENCE
<400> 8
   ggcaggtttg tcgacctcac t 21
<210> 9
   <211> 24
   <212> DNA
   <213> Artificial Sequence
<220>
   <223> Description of Artificial Sequence: MYOGENIN UP OLIGONUCLEOTIDE SEQUENCE
<400> 9
   caaccaggag gagcgcgatc tccg 24
<210> 10
   <211> 25
   <212> DNA
   <213> Artificial Sequence
<220>
   <223> Description of Artificial Sequence: MYOGENIN DOWN OLIGONUCLEOTIDE SEQUENCE
<400> 10
   aggcgctgtg ggagttgcat tcact 25
<210> 11
   <211> 27
   <212> DNA
   <213> Artificial Sequence
<220>
   <223> Description of Artificial Sequence: MYOD UP OLIGONUCLEOTIDE SEQUENCE
<400> 11
   gctctgatgg catgatggat tacagcg 27
<210> 12
   <211> 22
   <212> DNA
   <213> Artificial Sequence
<220>
   <223> Description of Artificial Sequence: MYOD DOWN OLIGONUCLEOTIDE SEQUENCE
<400> 12
   atgctggaca ggcagtcgag gc 22